# EUROPEAN PATENT SPECIFICATION

(11) **EP 3 414 572 B1**
(45) Date of publication and mention of the grant of the patent: **22.04.2020**
(21) Application number: 17704812.1
(22) Date of filing: 03.02.2017
(51) Int. Cl.: G01N 33/569, A61K 38/00, C12Q 1/68

(54) **TB BIOMARKERS**
TB-BIOMARKER
BIOMARQUEURS DE TB

(30) Priority: 09.02.2016 GB 201602305
(43) Date of publication of application: 19.12.2018
(73) Proprietor: United Kingdom Research and Innovation, Swindon SN2 1FL (GB); IP2IPO Innovations Limited, London EC4N 8AF (GB)
(72) Inventor: KAMPMANN, Beate, London WC1E 7HT (GB); TOGUN, Toyin, London WC1E 7HT (GB); HOGGART, Clive Julian, London W2 1PG (GB)
(74) Representative: Cooley (UK) LLP
(86) International application number: PCT/GB2017/050272
(87) International publication number: WO 2017/137727

(56) References cited:
- TOGUN TOYIN O ET AL: "No added value of interferon-[gamma] release to a prediction model for childhood tuberculosis.", THE EUROPEAN RESPIRATORY JOURNAL JAN 2016, vol. 47, no. 1, 22 October 2015 (2015-10-22), pages 223-232, XP008183758, ISSN: 1399-3003
- Unknown: "Bio-Plex ProTM Human Cytokine, Chemokine, and Growth Factor Assays", , 18 June 2014 (2014-06-18), pages 1-4, XP055355635, Retrieved from the Internet: URL:http://www.bio-rad.com/webroot/web/pdf /lsr/literature/Bulletin_5828.pdf [retrieved on 2017-03-16]
- Who: "Guidance for national tuberculosis programmes on the management of tuberculosis in children Second edition", , 5 May 2014 (2014-05-05), XP055355851, Retrieved from the Internet: URL:https://www.tballiance.org/sites/defau lt/files/child-resources/WHO_Guidance_nati onal_TB_programme_mngmt_TB_children.pdf [retrieved on 2017-03-17]
- I. WERGELAND ET AL: "IP-10 differentiates between active and latent tuberculosis irrespective of HIV status and declines during therapy", JOURNAL OF INFECTION., vol. 70, no. 4, 15 January 2015 (2015-01-15), pages 381-391, XP055355880, GB ISSN: 0163-4453, DOI: 10.1016/j.jinf.2014.12.019
- JOSÉ M. GUERRA-LASO ET AL: "Microarray analysis of Mycobacterium tuberculosis -infected monocytes reveals IL26 as a new candidate gene for tuberculosis susceptibility", IMMUNOLOGY, vol. 144, no. 2, 6 January 2015 (2015-01-06), pages 291-301, XP055355882, GB ISSN: 0019-2805, DOI: 10.1111/imm.12371

### Sequence Listing

### SEQUENCE LISTING

<110> Medical Research Council
   Imperial Innovations Limited
<120> TB BIOMARKERS
<130> P10280GBWO
<150> 1602305.3
   <151> 2016-02-09
<160> 30
<170> PatentIn version 3.5
<210> 1
   <211> 212
   <212> PRT
   <213> Homo sapiens
<400> 1
   Met Asn Ser Phe Ser Thr Ser Ala Phe Gly Pro Val Ala Phe Ser Leu
   1               5                   10                  15
   Gly Leu Leu Leu Val Leu Pro Ala Ala Phe Pro Ala Pro Val Pro Pro
               20                  25                  30
   Gly Glu Asp Ser Lys Asp Val Ala Ala Pro His Arg Gln Pro Leu Thr
           35                  40                  45
   Ser Ser Glu Arg Ile Asp Lys Gln Ile Arg Tyr Ile Leu Asp Gly Ile
       50                  55                  60
   Ser Ala Leu Arg Lys Glu Thr Cys Asn Lys Ser Asn Met Cys Glu Ser
   65                  70                  75                  80
   Ser Lys Glu Ala Leu Ala Glu Asn Asn Leu Asn Leu Pro Lys Met Ala
                   85                  90                  95
   Glu Lys Asp Gly Cys Phe Gln Ser Gly Phe Asn Glu Glu Thr Cys Leu
               100                 105                 110
   Val Lys Ile Ile Thr Gly Leu Leu Glu Phe Glu Val Tyr Leu Glu Tyr
           115                 120                 125
   Leu Gln Asn Arg Phe Glu Ser Ser Glu Glu Gln Ala Arg Ala Val Gln
       130                 135                 140
   Met Ser Thr Lys Val Leu Ile Gln Phe Leu Gln Lys Lys Ala Lys Asn
   145                 150                 155                 160
   Leu Asp Ala Ile Thr Thr Pro Asp Pro Thr Thr Asn Ala Ser Leu Leu
                   165                 170                 175
   Thr Lys Leu Gln Ala Gln Asn Gln Trp Leu Gln Asp Met Thr Thr His
               180                 185                 190
   Leu Ile Leu Arg Ser Phe Lys Glu Phe Leu Gln Ser Ser Leu Arg Ala
           195                 200                 205
   Leu Arg Gln Met
       210
<210> 2
   <211> 177
   <212> PRT
   <213> Homo sapiens
<400> 2
   Met Glu Ile Cys Arg Gly Leu Arg Ser His Leu Ile Thr Leu Leu Leu
   1               5                   10                  15
   Phe Leu Phe His Ser Glu Thr Ile Cys Arg Pro Ser Gly Arg Lys Ser
               20                  25                  30
   Ser Lys Met Gln Ala Phe Arg Ile Trp Asp Val Asn Gln Lys Thr Phe
           35                  40                  45
   Tyr Leu Arg Asn Asn Gln Leu Val Ala Gly Tyr Leu Gln Gly Pro Asn
       50                  55                  60
   Val Asn Leu Glu Glu Lys Ile Asp Val Val Pro Ile Glu Pro His Ala
   65                  70                  75                  80
   Leu Phe Leu Gly Ile His Gly Gly Lys Met Cys Leu Ser Cys Val Lys
                   85                  90                  95
   Ser Gly Asp Glu Thr Arg Leu Gln Leu Glu Ala Val Asn Ile Thr Asp
               100                 105                 110
   Leu Ser Glu Asn Arg Lys Gln Asp Lys Arg Phe Ala Phe Ile Arg Ser
           115                 120                 125
   Asp Ser Gly Pro Thr Thr Ser Phe Glu Ser Ala Ala Cys Pro Gly Trp
       130                 135                 140
   Phe Leu Cys Thr Ala Met Glu Ala Asp Gln Pro Val Ser Leu Thr Asn
   145                 150                 155                 160
   Met Pro Asp Glu Gly Val Met Val Thr Lys Phe Tyr Phe Gln Glu Asp
                   165                 170                 175
   Glu
<210> 3
   <211> 159
   <212> PRT
   <213> Homo sapiens
<400> 3
   Met Ala Leu Glu Thr Ile Cys Arg Pro Ser Gly Arg Lys Ser Ser Lys
   1               5                   10                  15
   Met Gln Ala Phe Arg Ile Trp Asp Val Asn Gln Lys Thr Phe Tyr Leu
               20                  25                  30
   Arg Asn Asn Gln Leu Val Ala Gly Tyr Leu Gln Gly Pro Asn Val Asn
           35                  40                  45
   Leu Glu Glu Lys Ile Asp Val Val Pro Ile Glu Pro His Ala Leu Phe
       50                  55                  60
   Leu Gly Ile His Gly Gly Lys Met Cys Leu Ser Cys Val Lys Ser Gly
   65                  70                  75                  80
   Asp Glu Thr Arg Leu Gln Leu Glu Ala Val Asn Ile Thr Asp Leu Ser
                   85                  90                  95
   Glu Asn Arg Lys Gln Asp Lys Arg Phe Ala Phe Ile Arg Ser Asp Ser
               100                 105                 110
   Gly Pro Thr Thr Ser Phe Glu Ser Ala Ala Cys Pro Gly Trp Phe Leu
           115                 120                 125
   Cys Thr Ala Met Glu Ala Asp Gln Pro Val Ser Leu Thr Asn Met Pro
       130                 135                 140
   Asp Glu Gly Val Met Val Thr Lys Phe Tyr Phe Gln Glu Asp Glu
   145                 150                 155
<210> 4
   <211> 180
   <212> PRT
   <213> Homo sapiens
<400> 4
   Met Ala Leu Ala Asp Leu Tyr Glu Glu Gly Gly Gly Gly Gly Gly Glu
   1               5                   10                  15
   Gly Glu Asp Asn Ala Asp Ser Lys Glu Thr Ile Cys Arg Pro Ser Gly
               20                  25                  30
   Arg Lys Ser Ser Lys Met Gln Ala Phe Arg Ile Trp Asp Val Asn Gln
           35                  40                  45
   Lys Thr Phe Tyr Leu Arg Asn Asn Gln Leu Val Ala Gly Tyr Leu Gln
       50                  55                  60
   Gly Pro Asn Val Asn Leu Glu Glu Lys Ile Asp Val Val Pro Ile Glu
   65                  70                  75                  80
   Pro His Ala Leu Phe Leu Gly Ile His Gly Gly Lys Met Cys Leu Ser
                   85                  90                  95
   Cys Val Lys Ser Gly Asp Glu Thr Arg Leu Gln Leu Glu Ala Val Asn
               100                 105                 110
   Ile Thr Asp Leu Ser Glu Asn Arg Lys Gln Asp Lys Arg Phe Ala Phe
           115                 120                 125
   Ile Arg Ser Asp Ser Gly Pro Thr Thr Ser Phe Glu Ser Ala Ala Cys
       130                 135                 140
   Pro Gly Trp Phe Leu Cys Thr Ala Met Glu Ala Asp Gln Pro Val Ser
   145                 150                 155                 160
   Leu Thr Asn Met Pro Asp Glu Gly Val Met Val Thr Lys Phe Tyr Phe
                   165                 170                 175
   Gln Glu Asp Glu
               180
<210> 5
   <211> 143
   <212> PRT
   <213> Homo sapiens
<400> 5
   Met Gln Ala Phe Arg Ile Trp Asp Val Asn Gln Lys Thr Phe Tyr Leu
   1               5                   10                  15
   Arg Asn Asn Gln Leu Val Ala Gly Tyr Leu Gln Gly Pro Asn Val Asn
               20                  25                  30
   Leu Glu Glu Lys Ile Asp Val Val Pro Ile Glu Pro His Ala Leu Phe
           35                  40                  45
   Leu Gly Ile His Gly Gly Lys Met Cys Leu Ser Cys Val Lys Ser Gly
       50                  55                  60
   Asp Glu Thr Arg Leu Gln Leu Glu Ala Val Asn Ile Thr Asp Leu Ser
   65                  70                  75                  80
   Glu Asn Arg Lys Gln Asp Lys Arg Phe Ala Phe Ile Arg Ser Asp Ser
                   85                  90                  95
   Gly Pro Thr Thr Ser Phe Glu Ser Ala Ala Cys Pro Gly Trp Phe Leu
               100                 105                 110
   Cys Thr Ala Met Glu Ala Asp Gln Pro Val Ser Leu Thr Asn Met Pro
           115                 120                 125
   Asp Glu Gly Val Met Val Thr Lys Phe Tyr Phe Gln Glu Asp Glu
       130                 135                 140
<210> 6
   <211> 177
   <212> PRT
   <213> Homo sapiens
<400> 6
   Met Phe His Val Ser Phe Arg Tyr Ile Phe Gly Leu Pro Pro Leu Ile
   1               5                   10                  15
   Leu Val Leu Leu Pro Val Ala Ser Ser Asp Cys Asp Ile Glu Gly Lys
               20                  25                  30
   Asp Gly Lys Gln Tyr Glu Ser Val Leu Met Val Ser Ile Asp Gln Leu
           35                  40                  45
   Leu Asp Ser Met Lys Glu Ile Gly Ser Asn Cys Leu Asn Asn Glu Phe
       50                  55                  60
   Asn Phe Phe Lys Arg His Ile Cys Asp Ala Asn Lys Glu Gly Met Phe
   65                  70                  75                  80
   Leu Phe Arg Ala Ala Arg Lys Leu Arg Gln Phe Leu Lys Met Asn Ser
                   85                  90                  95
   Thr Gly Asp Phe Asp Leu His Leu Leu Lys Val Ser Glu Gly Thr Thr
               100                 105                 110
   Ile Leu Leu Asn Cys Thr Gly Gln Val Lys Gly Arg Lys Pro Ala Ala
           115                 120                 125
   Leu Gly Glu Ala Gln Pro Thr Lys Ser Leu Glu Glu Asn Lys Ser Leu
       130                 135                 140
   Lys Glu Gln Lys Lys Leu Asn Asp Leu Cys Phe Leu Lys Arg Leu Leu
   145                 150                 155                 160
   Gln Glu Ile Lys Thr Cys Trp Asn Lys Ile Leu Met Gly Thr Lys Glu
                   165                 170                 175
   His
<210> 7
   <211> 133
   <212> PRT
   <213> Homo sapiens
<400> 7
   Met Phe His Val Ser Phe Arg Tyr Ile Phe Gly Leu Pro Pro Leu Ile
   1               5                   10                  15
   Leu Val Leu Leu Pro Val Ala Ser Ser Asp Cys Asp Ile Glu Gly Lys
               20                  25                  30
   Asp Gly Lys Gln Tyr Glu Ser Val Leu Met Val Ser Ile Asp Gln Leu
           35                  40                  45
   Leu Asp Ser Met Lys Glu Ile Gly Ser Asn Cys Leu Asn Asn Glu Phe
       50                  55                  60
   Asn Phe Phe Lys Arg His Ile Cys Asp Ala Asn Lys Val Lys Gly Arg
   65                  70                  75                  80
   Lys Pro Ala Ala Leu Gly Glu Ala Gln Pro Thr Lys Ser Leu Glu Glu
                   85                  90                  95
   Asn Lys Ser Leu Lys Glu Gln Lys Lys Leu Asn Asp Leu Cys Phe Leu
               100                 105                 110
   Lys Arg Leu Leu Gln Glu Ile Lys Thr Cys Trp Asn Lys Ile Leu Met
           115                 120                 125
   Gly Thr Lys Glu His
       130
<210> 8
   <211> 64
   <212> PRT
   <213> Homo sapiens
<400> 8
   Met Lys Glu Ile Gly Ser Asn Cys Leu Asn Asn Glu Phe Asn Phe Phe
   1               5                   10                  15
   Lys Arg His Ile Cys Asp Ala Asn Lys Glu Glu Asn Lys Ser Leu Lys
               20                  25                  30
   Glu Gln Lys Lys Leu Asn Asp Leu Cys Phe Leu Lys Arg Leu Leu Gln
           35                  40                  45
   Glu Ile Lys Thr Cys Trp Asn Lys Ile Leu Met Gly Thr Lys Glu His
       50                  55                  60
<210> 9
   <211> 99
   <212> PRT
   <213> Homo sapiens
<400> 9
   Met Thr Ser Lys Leu Ala Val Ala Leu Leu Ala Ala Phe Leu Ile Ser
   1               5                   10                  15
   Ala Ala Leu Cys Glu Gly Ala Val Leu Pro Arg Ser Ala Lys Glu Leu
               20                  25                  30
   Arg Cys Gln Cys Ile Lys Thr Tyr Ser Lys Pro Phe His Pro Lys Phe
           35                  40                  45
   Ile Lys Glu Leu Arg Val Ile Glu Ser Gly Pro His Cys Ala Asn Thr
       50                  55                  60
   Glu Ile Ile Val Lys Leu Ser Asp Gly Arg Glu Leu Cys Leu Asp Pro
   65                  70                  75                  80
   Lys Glu Asn Trp Val Gln Arg Val Val Glu Lys Phe Leu Lys Arg Ala
                   85                  90                  95
   Glu Asn Ser
<210> 10
   <211> 102
   <212> PRT
   <213> Homo sapiens
<400> 10
   Met Thr Ser Lys Leu Ala Val Ala Leu Leu Ala Ala Phe Leu Ile Ser
   1 5 10 15
   Ala Ala Leu Cys Glu Gly Ala Val Leu Pro Arg Ser Ala Lys Glu Leu
   20 25 30
   Arg Cys Gln Cys Ile Lys Thr Tyr Ser Lys Pro Phe His Pro Lys Phe
   35 40 45
   Ile Lys Glu Leu Arg Val Ile Glu Ser Gly Pro His Cys Ala Asn Thr
   50 55 60
   Glu Ile Ile Val Lys Leu Ser Asp Gly Arg Glu Leu Cys Leu Asp Pro
   65 70 75 80
   Lys Glu Asn Trp Val Gln Arg Val Val Glu Lys Ala Glu Val Pro Glu
   85 90 95
   Asn Arg Gly Met Asp Ser
   100
<210> 11
   <211> 328
   <212> PRT
   <213> Homo sapiens
<400> 11
   Met Cys His Gln Gln Leu Val Ile Ser Trp Phe Ser Leu Val Phe Leu
   1 5 10 15
   Ala Ser Pro Leu Val Ala Ile Trp Glu Leu Lys Lys Asp Val Tyr Val
   20 25 30
   Val Glu Leu Asp Trp Tyr Pro Asp Ala Pro Gly Glu Met Val Val Leu
   35 40 45
   Thr Cys Asp Thr Pro Glu Glu Asp Gly Ile Thr Trp Thr Leu Asp Gln
   50 55 60
   Ser Ser Glu Val Leu Gly Ser Gly Lys Thr Leu Thr Ile Gln Val Lys
   65 70 75 80
   Glu Phe Gly Asp Ala Gly Gln Tyr Thr Cys His Lys Gly Gly Glu Val
   85 90 95
   Leu Ser His Ser Leu Leu Leu Leu His Lys Lys Glu Asp Gly Ile Trp
   100 105 110
   Ser Thr Asp Ile Leu Lys Asp Gln Lys Glu Pro Lys Asn Lys Thr Phe
           115                 120                 125
   Leu Arg Cys Glu Ala Lys Asn Tyr Ser Gly Arg Phe Thr Cys Trp Trp
       130                 135                 140
   Leu Thr Thr Ile Ser Thr Asp Leu Thr Phe Ser Val Lys Ser Ser Arg
   145                 150                 155                 160
   Gly Ser Ser Asp Pro Gln Gly Val Thr Cys Gly Ala Ala Thr Leu Ser
                   165                 170                 175
   Ala Glu Arg Val Arg Gly Asp Asn Lys Glu Tyr Glu Tyr Ser Val Glu
               180                 185                 190
   Cys Gln Glu Asp Ser Ala Cys Pro Ala Ala Glu Glu Ser Leu Pro Ile
           195                 200                 205
   Glu Val Met Val Asp Ala Val His Lys Leu Lys Tyr Glu Asn Tyr Thr
       210                 215                 220
   Ser Ser Phe Phe Ile Arg Asp Ile Ile Lys Pro Asp Pro Pro Lys Asn
   225                 230                 235                 240
   Leu Gln Leu Lys Pro Leu Lys Asn Ser Arg Gln Val Glu Val Ser Trp
                   245                 250                 255
   Glu Tyr Pro Asp Thr Trp Ser Thr Pro His Ser Tyr Phe Ser Leu Thr
               260                 265                 270
   Phe Cys Val Gln Val Gln Gly Lys Ser Lys Arg Glu Lys Lys Asp Arg
           275                 280                 285
   Val Phe Thr Asp Lys Thr Ser Ala Thr Val Ile Cys Arg Lys Asn Ala
       290                 295                 300
   Ser Ile Ser Val Arg Ala Gln Asp Arg Tyr Tyr Ser Ser Ser Trp Ser
   305                 310                 315                 320
   Glu Trp Ala Ser Val Pro Cys Ser
                   325
<210> 12
   <211> 288
   <212> PRT
   <213> Homo sapiens
<400> 12
   Met Val Gly Val Gly Gly Gly Asp Val Glu Asp Val Thr Pro Arg Pro
   1               5                   10                  15
   Gly Gly Cys Gln Ile Ser Gly Arg Gly Ala Arg Gly Cys Asn Gly Ile
               20                  25                  30
   Pro Gly Ala Ala Ala Trp Glu Ala Ala Leu Pro Arg Arg Arg Pro Arg
           35                  40                  45
   Arg His Pro Ser Val Asn Pro Arg Ser Arg Ala Ala Gly Ser Pro Arg
       50                  55                  60
   Thr Arg Gly Arg Arg Thr Glu Glu Arg Pro Ser Gly Ser Arg Leu Gly
   65                  70                  75                  80
   Asp Arg Gly Arg Gly Arg Ala Leu Pro Gly Gly Arg Leu Gly Gly Arg
                   85                  90                  95
   Gly Arg Gly Arg Ala Pro Glu Arg Val Gly Gly Arg Gly Arg Gly Arg
               100                 105                 110
   Gly Thr Ala Ala Pro Arg Ala Ala Pro Ala Ala Arg Gly Ser Arg Pro
           115                 120                 125
   Gly Pro Ala Gly Thr Met Ala Ala Gly Ser Ile Thr Thr Leu Pro Ala
       130                 135                 140
   Leu Pro Glu Asp Gly Gly Ser Gly Ala Phe Pro Pro Gly His Phe Lys
   145                 150                 155                 160
   Asp Pro Lys Arg Leu Tyr Cys Lys Asn Gly Gly Phe Phe Leu Arg Ile
                   165                 170                 175
   His Pro Asp Gly Arg Val Asp Gly Val Arg Glu Lys Ser Asp Pro His
               180                 185                 190
   Ile Lys Leu Gln Leu Gln Ala Glu Glu Arg Gly Val Val Ser Ile Lys
           195                 200                 205
   Gly Val Cys Ala Asn Arg Tyr Leu Ala Met Lys Glu Asp Gly Arg Leu
       210                 215                 220
   Leu Ala Ser Lys Cys Val Thr Asp Glu Cys Phe Phe Phe Glu Arg Leu
   225                 230                 235                 240
   Glu Ser Asn Asn Tyr Asn Thr Tyr Arg Ser Arg Lys Tyr Thr Ser Trp
                   245                 250                 255
   Tyr Val Ala Leu Lys Arg Thr Gly Gln Tyr Lys Leu Gly Ser Lys Thr
   260 265 270
   Gly Pro Gly Gln Lys Ala Ile Leu Phe Leu Pro Met Ser Ala Lys Ser
   275 280 285
<210> 13
   <211> 98
   <212> PRT
   <213> Homo sapiens
<400> 13
   Met Asn Gln Thr Ala Ile Leu Ile Cys Cys Leu Ile Phe Leu Thr Leu
   1 5 10 15
   Ser Gly Ile Gln Gly Val Pro Leu Ser Arg Thr Val Arg Cys Thr Cys
   20 25 30
   Ile Ser Ile Ser Asn Gln Pro Val Asn Pro Arg Ser Leu Glu Lys Leu
   35 40 45
   Glu Ile Ile Pro Ala Ser Gln Phe Cys Pro Arg Val Glu Ile Ile Ala
   50 55 60
   Thr Met Lys Lys Lys Gly Glu Lys Arg Cys Leu Asn Pro Glu Ser Lys
   65 70 75 80
   Ala Ile Lys Asn Leu Leu Lys Ala Val Ser Lys Glu Arg Ser Lys Arg
   85 90 95
   Ser Pro
<210> 14
   <211> 232
   <212> PRT
   <213> Homo sapiens
<400> 14
   Met Asn Phe Leu Leu Ser Trp Val His Trp Ser Leu Ala Leu Leu Leu
   1 5 10 15
   Tyr Leu His His Ala Lys Trp Ser Gln Ala Ala Pro Met Ala Glu Gly
   20 25 30
   Gly Gly Gln Asn His His Glu Val Val Lys Phe Met Asp Val Tyr Gln
   35 40 45
   Arg Ser Tyr Cys His Pro Ile Glu Thr Leu Val Asp Ile Phe Gln Glu
       50                  55                  60
   Tyr Pro Asp Glu Ile Glu Tyr Ile Phe Lys Pro Ser Cys Val Pro Leu
   65                  70                  75                  80
   Met Arg Cys Gly Gly Cys Cys Asn Asp Glu Gly Leu Glu Cys Val Pro
                   85                  90                  95
   Thr Glu Glu Ser Asn Ile Thr Met Gln Ile Met Arg Ile Lys Pro His
               100                 105                 110
   Gln Gly Gln His Ile Gly Glu Met Ser Phe Leu Gln His Asn Lys Cys
           115                 120                 125
   Glu Cys Arg Pro Lys Lys Asp Arg Ala Arg Gln Glu Lys Lys Ser Val
       130                 135                 140
   Arg Gly Lys Gly Lys Gly Gln Lys Arg Lys Arg Lys Lys Ser Arg Tyr
   145                 150                 155                 160
   Lys Ser Trp Ser Val Tyr Val Gly Ala Arg Cys Cys Leu Met Pro Trp
                   165                 170                 175
   Ser Leu Pro Gly Pro His Pro Cys Gly Pro Cys Ser Glu Arg Arg Lys
               180                 185                 190
   His Leu Phe Val Gln Asp Pro Gln Thr Cys Lys Cys Ser Cys Lys Asn
           195                 200                 205
   Thr Asp Ser Arg Cys Lys Ala Arg Gln Leu Glu Leu Asn Glu Arg Thr
       210                 215                 220
   Cys Arg Cys Asp Lys Pro Arg Arg
   225                 230
<210> 15
   <211> 215
   <212> PRT
   <213> Homo sapiens
<400> 15
   Met Asn Phe Leu Leu Ser Trp Val His Trp Ser Leu Ala Leu Leu Leu
   1               5                   10                  15
   Tyr Leu His His Ala Lys Trp Ser Gln Ala Ala Pro Met Ala Glu Gly
               20                  25                  30
   Gly Gly Gln Asn His His Glu Val Val Lys Phe Met Asp Val Tyr Gln
   35 40 45
   Arg Ser Tyr Cys His Pro Ile Glu Thr Leu Val Asp Ile Phe Gln Glu
   50 55 60
   Tyr Pro Asp Glu Ile Glu Tyr Ile Phe Lys Pro Ser Cys Val Pro Leu
   65 70 75 80
   Met Arg Cys Gly Gly Cys Cys Asn Asp Glu Gly Leu Glu Cys Val Pro
   85 90 95
   Thr Glu Glu Ser Asn Ile Thr Met Gln Ile Met Arg Ile Lys Pro His
   100 105 110
   Gln Gly Gln His Ile Gly Glu Met Ser Phe Leu Gln His Asn Lys Cys
   115 120 125
   Glu Cys Arg Pro Lys Lys Asp Arg Ala Arg Gln Glu Lys Lys Ser Val
   130 135 140
   Arg Gly Lys Gly Lys Gly Gln Lys Arg Lys Arg Lys Lys Ser Arg Tyr
   145 150 155 160
   Lys Ser Trp Ser Val Pro Cys Gly Pro Cys Ser Glu Arg Arg Lys His
   165 170 175
   Leu Phe Val Gln Asp Pro Gln Thr Cys Lys Cys Ser Cys Lys Asn Thr
   180 185 190
   Asp Ser Arg Cys Lys Ala Arg Gln Leu Glu Leu Asn Glu Arg Thr Cys
   195 200 205
   Arg Cys Asp Lys Pro Arg Arg
   210 215
<210> 16
   <211> 209
   <212> PRT
   <213> Homo sapiens
<400> 16
   Met Asn Phe Leu Leu Ser Trp Val His Trp Ser Leu Ala Leu Leu Leu
   1 5 10 15
   Tyr Leu His His Ala Lys Trp Ser Gln Ala Ala Pro Met Ala Glu Gly
   20 25 30
   Gly Gly Gln Asn His His Glu Val Val Lys Phe Met Asp Val Tyr Gln
           35                  40                  45
   Arg Ser Tyr Cys His Pro Ile Glu Thr Leu Val Asp Ile Phe Gln Glu
       50                  55                  60
   Tyr Pro Asp Glu Ile Glu Tyr Ile Phe Lys Pro Ser Cys Val Pro Leu
   65                  70                  75                  80
   Met Arg Cys Gly Gly Cys Cys Asn Asp Glu Gly Leu Glu Cys Val Pro
                   85                  90                  95
   Thr Glu Glu Ser Asn Ile Thr Met Gln Ile Met Arg Ile Lys Pro His
               100                 105                 110
   Gln Gly Gln His Ile Gly Glu Met Ser Phe Leu Gln His Asn Lys Cys
           115                 120                 125
   Glu Cys Arg Pro Lys Lys Asp Arg Ala Arg Gln Glu Lys Lys Ser Val
       130                 135                 140
   Arg Gly Lys Gly Lys Gly Gln Lys Arg Lys Arg Lys Lys Ser Arg Pro
   145                 150                 155                 160
   Cys Gly Pro Cys Ser Glu Arg Arg Lys His Leu Phe Val Gln Asp Pro
                   165                 170                 175
   Gln Thr Cys Lys Cys Ser Cys Lys Asn Thr Asp Ser Arg Cys Lys Ala
               180                 185                 190
   Arg Gln Leu Glu Leu Asn Glu Arg Thr Cys Arg Cys Asp Lys Pro Arg
           195                 200                 205
   Arg
<210> 17
   <211> 191
   <212> PRT
   <213> Homo sapiens
<400> 17
   Met Asn Phe Leu Leu Ser Trp Val His Trp Ser Leu Ala Leu Leu Leu
   1               5                   10                  15
   Tyr Leu His His Ala Lys Trp Ser Gln Ala Ala Pro Met Ala Glu Gly
               20                  25                  30
   Gly Gly Gln Asn His His Glu Val Val Lys Phe Met Asp Val Tyr Gln
   35 40 45
   Arg Ser Tyr Cys His Pro Ile Glu Thr Leu Val Asp Ile Phe Gln Glu
   50 55 60
   Tyr Pro Asp Glu Ile Glu Tyr Ile Phe Lys Pro Ser Cys Val Pro Leu
   65 70 75 80
   Met Arg Cys Gly Gly Cys Cys Asn Asp Glu Gly Leu Glu Cys Val Pro
   85 90 95
   Thr Glu Glu Ser Asn Ile Thr Met Gln Ile Met Arg Ile Lys Pro His
   100 105 110
   Gln Gly Gln His Ile Gly Glu Met Ser Phe Leu Gln His Asn Lys Cys
   115 120 125
   Glu Cys Arg Pro Lys Lys Asp Arg Ala Arg Gln Glu Asn Pro Cys Gly
   130 135 140
   Pro Cys Ser Glu Arg Arg Lys His Leu Phe Val Gln Asp Pro Gln Thr
   145 150 155 160
   Cys Lys Cys Ser Cys Lys Asn Thr Asp Ser Arg Cys Lys Ala Arg Gln
   165 170 175
   Leu Glu Leu Asn Glu Arg Thr Cys Arg Cys Asp Lys Pro Arg Arg
   180 185 190
<210> 18
   <211> 174
   <212> PRT
   <213> Homo sapiens
<400> 18
   Met Asn Phe Leu Leu Ser Trp Val His Trp Ser Leu Ala Leu Leu Leu
   1 5 10 15
   Tyr Leu His His Ala Lys Trp Ser Gln Ala Ala Pro Met Ala Glu Gly
   20 25 30
   Gly Gly Gln Asn His His Glu Val Val Lys Phe Met Asp Val Tyr Gln
   35 40 45
   Arg Ser Tyr Cys His Pro Ile Glu Thr Leu Val Asp Ile Phe Gln Glu
   50 55 60
   Tyr Pro Asp Glu Ile Glu Tyr Ile Phe Lys Pro Ser Cys Val Pro Leu
   65 70 75 80
   Met Arg Cys Gly Gly Cys Cys Asn Asp Glu Gly Leu Glu Cys Val Pro
   85 90 95
   Thr Glu Glu Ser Asn Ile Thr Met Gln Ile Met Arg Ile Lys Pro His
   100 105 110
   Gln Gly Gln His Ile Gly Glu Met Ser Phe Leu Gln His Asn Lys Cys
   115 120 125
   Glu Cys Arg Pro Lys Lys Asp Arg Ala Arg Gln Glu Asn Pro Cys Gly
   130 135 140
   Pro Cys Ser Glu Arg Arg Lys His Leu Phe Val Gln Asp Pro Gln Thr
   145 150 155 160
   Cys Lys Cys Ser Cys Lys Asn Thr Asp Ser Arg Cys Lys Met
   165 170
<210> 19
   <211> 171
   <212> PRT
   <213> Homo sapiens
<400> 19
   Met Asn Phe Leu Leu Ser Trp Val His Trp Ser Leu Ala Leu Leu Leu
   1 5 10 15
   Tyr Leu His His Ala Lys Trp Ser Gln Ala Ala Pro Met Ala Glu Gly
   20 25 30
   Gly Gly Gln Asn His His Glu Val Val Lys Phe Met Asp Val Tyr Gln
   35 40 45
   Arg Ser Tyr Cys His Pro Ile Glu Thr Leu Val Asp Ile Phe Gln Glu
   50 55 60
   Tyr Pro Asp Glu Ile Glu Tyr Ile Phe Lys Pro Ser Cys Val Pro Leu
   65 70 75 80
   Met Arg Cys Gly Gly Cys Cys Asn Asp Glu Gly Leu Glu Cys Val Pro
   85 90 95
   Thr Glu Glu Ser Asn Ile Thr Met Gln Ile Met Arg Ile Lys Pro His
   100 105 110
   Gln Gly Gln His Ile Gly Glu Met Ser Phe Leu Gln His Asn Lys Cys
           115                 120                 125
   Glu Cys Arg Pro Lys Lys Asp Arg Ala Arg Gln Glu Lys Lys Ser Val
       130                 135                 140
   Arg Gly Lys Gly Lys Gly Gln Lys Arg Lys Arg Lys Lys Ser Arg Tyr
   145                 150                 155                 160
   Lys Ser Trp Ser Val Cys Asp Lys Pro Arg Arg
                   165                 170
<210> 20
   <211> 191
   <212> PRT
   <213> Homo sapiens
<400> 20
   Met Asn Phe Leu Leu Ser Trp Val His Trp Ser Leu Ala Leu Leu Leu
   1               5                   10                  15
   Tyr Leu His His Ala Lys Trp Ser Gln Ala Ala Pro Met Ala Glu Gly
               20                  25                  30
   Gly Gly Gln Asn His His Glu Val Val Lys Phe Met Asp Val Tyr Gln
           35                  40                  45
   Arg Ser Tyr Cys His Pro Ile Glu Thr Leu Val Asp Ile Phe Gln Glu
       50                  55                  60
   Tyr Pro Asp Glu Ile Glu Tyr Ile Phe Lys Pro Ser Cys Val Pro Leu
   65                  70                  75                  80
   Met Arg Cys Gly Gly Cys Cys Asn Asp Glu Gly Leu Glu Cys Val Pro
                   85                  90                  95
   Thr Glu Glu Ser Asn Ile Thr Met Gln Ile Met Arg Ile Lys Pro His
               100                 105                 110
   Gln Gly Gln His Ile Gly Glu Met Ser Phe Leu Gln His Asn Lys Cys
           115                 120                 125
   Glu Cys Arg Pro Lys Lys Asp Arg Ala Arg Gln Glu Asn Pro Cys Gly
       130                 135                 140
   Pro Cys Ser Glu Arg Arg Lys His Leu Phe Val Gln Asp Pro Gln Thr
   145                 150                 155                 160
   Cys Lys Cys Ser Cys Lys Asn Thr Asp Ser Arg Cys Lys Ala Arg Gln
                   165                 170                 175
   Leu Glu Leu Asn Glu Arg Thr Cys Arg Ser Leu Thr Arg Lys Asp
               180                 185                 190
<210> 21
   <211> 147
   <212> PRT
   <213> Homo sapiens
<400> 21
   Met Asn Phe Leu Leu Ser Trp Val His Trp Ser Leu Ala Leu Leu Leu
   1               5                   10                  15
   Tyr Leu His His Ala Lys Trp Ser Gln Ala Ala Pro Met Ala Glu Gly
               20                  25                  30
   Gly Gly Gln Asn His His Glu Val Val Lys Phe Met Asp Val Tyr Gln
           35                  40                  45
   Arg Ser Tyr Cys His Pro Ile Glu Thr Leu Val Asp Ile Phe Gln Glu
       50                  55                  60
   Tyr Pro Asp Glu Ile Glu Tyr Ile Phe Lys Pro Ser Cys Val Pro Leu
   65                  70                  75                  80
   Met Arg Cys Gly Gly Cys Cys Asn Asp Glu Gly Leu Glu Cys Val Pro
                   85                  90                  95
   Thr Glu Glu Ser Asn Ile Thr Met Gln Ile Met Arg Ile Lys Pro His
               100                 105                 110
   Gln Gly Gln His Ile Gly Glu Met Ser Phe Leu Gln His Asn Lys Cys
           115                 120                 125
   Glu Cys Arg Pro Lys Lys Asp Arg Ala Arg Gln Glu Lys Cys Asp Lys
       130                 135                 140
   Pro Arg Arg
   145
<210> 22
   <211> 137
   <212> PRT
   <213> Homo sapiens
<400> 22
   Met Asn Phe Leu Leu Ser Trp Val His Trp Ser Leu Ala Leu Leu Leu
   1               5                   10                  15
   Tyr Leu His His Ala Lys Trp Ser Gln Ala Ala Pro Met Ala Glu Gly
               20                  25                  30
   Gly Gly Gln Asn His His Glu Val Val Lys Phe Met Asp Val Tyr Gln
           35                  40                  45
   Arg Ser Tyr Cys His Pro Ile Glu Thr Leu Val Asp Ile Phe Gln Glu
       50                  55                  60
   Tyr Pro Asp Glu Ile Glu Tyr Ile Phe Lys Pro Ser Cys Val Pro Leu
   65                  70                  75                  80
   Met Arg Cys Gly Gly Cys Cys Asn Asp Glu Gly Leu Glu Cys Val Pro
                   85                  90                  95
   Thr Glu Glu Ser Asn Ile Thr Met Gln Ile Met Arg Ile Lys Pro His
               100                 105                 110
   Gln Gly Gln His Ile Gly Glu Met Ser Phe Leu Gln His Asn Lys Cys
           115                 120                 125
   Glu Cys Arg Cys Asp Lys Pro Arg Arg
       130                 135
<210> 23
   <211> 371
   <212> PRT
   <213> Homo sapiens
<400> 23
   Met Thr Asp Arg Gln Thr Asp Thr Ala Pro Ser Pro Ser Tyr His Leu
   1               5                   10                  15
   Leu Pro Gly Arg Arg Arg Thr Val Asp Ala Ala Ala Ser Arg Gly Gln
               20                  25                  30
   Gly Pro Glu Pro Ala Pro Gly Gly Gly Val Glu Gly Val Gly Ala Arg
           35                  40                  45
   Gly Val Ala Leu Lys Leu Phe Val Gln Leu Leu Gly Cys Ser Arg Phe
       50                  55                  60
   Gly Gly Ala Val Val Arg Ala Gly Glu Ala Glu Pro Ser Gly Ala Ala
   65                  70                  75                  80
   Arg Ser Ala Ser Ser Gly Arg Glu Glu Pro Gln Pro Glu Glu Gly Glu
                   85                  90                  95
   Glu Glu Glu Glu Lys Glu Glu Glu Arg Gly Pro Gln Trp Arg Leu Gly
               100                 105                 110
   Ala Arg Lys Pro Gly Ser Trp Thr Gly Glu Ala Ala Val Cys Ala Asp
           115                 120                 125
   Ser Ala Pro Ala Ala Arg Ala Pro Gln Ala Leu Ala Arg Ala Ser Gly
       130                 135                 140
   Arg Gly Gly Arg Val Ala Arg Arg Gly Ala Glu Glu Ser Gly Pro Pro
   145                 150                 155                 160
   His Ser Pro Ser Arg Arg Gly Ser Ala Ser Arg Ala Gly Pro Gly Arg
                   165                 170                 175
   Ala Ser Glu Thr Met Asn Phe Leu Leu Ser Trp Val His Trp Ser Leu
               180                 185                 190
   Ala Leu Leu Leu Tyr Leu His His Ala Lys Trp Ser Gln Ala Ala Pro
           195                 200                 205
   Met Ala Glu Gly Gly Gly Gln Asn His His Glu Val Val Lys Phe Met
       210                 215                 220
   Asp Val Tyr Gln Arg Ser Tyr Cys His Pro Ile Glu Thr Leu Val Asp
   225                 230                 235                 240
   Ile Phe Gln Glu Tyr Pro Asp Glu Ile Glu Tyr Ile Phe Lys Pro Ser
                   245                 250                 255
   Cys Val Pro Leu Met Arg Cys Gly Gly Cys Cys Asn Asp Glu Gly Leu
               260                 265                 270
   Glu Cys Val Pro Thr Glu Glu Ser Asn Ile Thr Met Gln Ile Met Arg
           275                 280                 285
   Ile Lys Pro His Gln Gly Gln His Ile Gly Glu Met Ser Phe Leu Gln
       290                 295                 300
   His Asn Lys Cys Glu Cys Arg Pro Lys Lys Asp Arg Ala Arg Gln Glu
   305                 310                 315                 320
   Asn Pro Cys Gly Pro Cys Ser Glu Arg Arg Lys His Leu Phe Val Gln
                   325                 330                 335
   Asp Pro Gln Thr Cys Lys Cys Ser Cys Lys Asn Thr Asp Ser Arg Cys
   340 345 350
   Lys Ala Arg Gln Leu Glu Leu Asn Glu Arg Thr Cys Arg Cys Asp Lys
   355 360 365
   Pro Arg Arg
   370
<210> 24
   <211> 327
   <212> PRT
   <213> Homo sapiens
<400> 24
   Met Thr Asp Arg Gln Thr Asp Thr Ala Pro Ser Pro Ser Tyr His Leu
   1 5 10 15
   Leu Pro Gly Arg Arg Arg Thr Val Asp Ala Ala Ala Ser Arg Gly Gln
   20 25 30
   Gly Pro Glu Pro Ala Pro Gly Gly Gly Val Glu Gly Val Gly Ala Arg
   35 40 45
   Gly Val Ala Leu Lys Leu Phe Val Gln Leu Leu Gly Cys Ser Arg Phe
   50 55 60
   Gly Gly Ala Val Val Arg Ala Gly Glu Ala Glu Pro Ser Gly Ala Ala
   65 70 75 80
   Arg Ser Ala Ser Ser Gly Arg Glu Glu Pro Gln Pro Glu Glu Gly Glu
   85 90 95
   Glu Glu Glu Glu Lys Glu Glu Glu Arg Gly Pro Gln Trp Arg Leu Gly
   100 105 110
   Ala Arg Lys Pro Gly Ser Trp Thr Gly Glu Ala Ala Val Cys Ala Asp
   115 120 125
   Ser Ala Pro Ala Ala Arg Ala Pro Gln Ala Leu Ala Arg Ala Ser Gly
   130 135 140
   Arg Gly Gly Arg Val Ala Arg Arg Gly Ala Glu Glu Ser Gly Pro Pro
   145 150 155 160
   His Ser Pro Ser Arg Arg Gly Ser Ala Ser Arg Ala Gly Pro Gly Arg
   165 170 175
   Ala Ser Glu Thr Met Asn Phe Leu Leu Ser Trp Val His Trp Ser Leu
                   180                 185                 190
   Ala Leu Leu Leu Tyr Leu His His Ala Lys Trp Ser Gln Ala Ala Pro
           195                 200                 205
   Met Ala Glu Gly Gly Gly Gln Asn His His Glu Val Val Lys Phe Met
       210                 215                 220
   Asp Val Tyr Gln Arg Ser Tyr Cys His Pro Ile Glu Thr Leu Val Asp
   225                 230                 235                 240
   Ile Phe Gln Glu Tyr Pro Asp Glu Ile Glu Tyr Ile Phe Lys Pro Ser
                   245                 250                 255
   Cys Val Pro Leu Met Arg Cys Gly Gly Cys Cys Asn Asp Glu Gly Leu
               260                 265                 270
   Glu Cys Val Pro Thr Glu Glu Ser Asn Ile Thr Met Gln Ile Met Arg
           275                 280                 285
   Ile Lys Pro His Gln Gly Gln His Ile Gly Glu Met Ser Phe Leu Gln
       290                 295                 300
   His Asn Lys Cys Glu Cys Arg Pro Lys Lys Asp Arg Ala Arg Gln Glu
   305                 310                 315                 320
   Lys Cys Asp Lys Pro Arg Arg
                   325
<210> 25
   <211> 395
   <212> PRT
   <213> Homo sapiens
<400> 25
   Met Thr Asp Arg Gln Thr Asp Thr Ala Pro Ser Pro Ser Tyr His Leu
   1               5                   10                  15
   Leu Pro Gly Arg Arg Arg Thr Val Asp Ala Ala Ala Ser Arg Gly Gln
               20                  25                  30
   Gly Pro Glu Pro Ala Pro Gly Gly Gly Val Glu Gly Val Gly Ala Arg
           35                  40                  45
   Gly Val Ala Leu Lys Leu Phe Val Gln Leu Leu Gly Cys Ser Arg Phe
       50                  55                  60
   Gly Gly Ala Val Val Arg Ala Gly Glu Ala Glu Pro Ser Gly Ala Ala
   65 70 75 80
   Arg Ser Ala Ser Ser Gly Arg Glu Glu Pro Gln Pro Glu Glu Gly Glu
   85 90 95
   Glu Glu Glu Glu Lys Glu Glu Glu Arg Gly Pro Gln Trp Arg Leu Gly
   100 105 110
   Ala Arg Lys Pro Gly Ser Trp Thr Gly Glu Ala Ala Val Cys Ala Asp
   115 120 125
   Ser Ala Pro Ala Ala Arg Ala Pro Gln Ala Leu Ala Arg Ala Ser Gly
   130 135 140
   Arg Gly Gly Arg Val Ala Arg Arg Gly Ala Glu Glu Ser Gly Pro Pro
   145 150 155 160
   His Ser Pro Ser Arg Arg Gly Ser Ala Ser Arg Ala Gly Pro Gly Arg
   165 170 175
   Ala Ser Glu Thr Met Asn Phe Leu Leu Ser Trp Val His Trp Ser Leu
   180 185 190
   Ala Leu Leu Leu Tyr Leu His His Ala Lys Trp Ser Gln Ala Ala Pro
   195 200 205
   Met Ala Glu Gly Gly Gly Gln Asn His His Glu Val Val Lys Phe Met
   210 215 220
   Asp Val Tyr Gln Arg Ser Tyr Cys His Pro Ile Glu Thr Leu Val Asp
   225 230 235 240
   Ile Phe Gln Glu Tyr Pro Asp Glu Ile Glu Tyr Ile Phe Lys Pro Ser
   245 250 255
   Cys Val Pro Leu Met Arg Cys Gly Gly Cys Cys Asn Asp Glu Gly Leu
   260 265 270
   Glu Cys Val Pro Thr Glu Glu Ser Asn Ile Thr Met Gln Ile Met Arg
   275 280 285
   Ile Lys Pro His Gln Gly Gln His Ile Gly Glu Met Ser Phe Leu Gln
   290 295 300
   His Asn Lys Cys Glu Cys Arg Pro Lys Lys Asp Arg Ala Arg Gln Glu
   305 310 315 320
   Lys Lys Ser Val Arg Gly Lys Gly Lys Gly Gln Lys Arg Lys Arg Lys
                   325                 330                 335
   Lys Ser Arg Tyr Lys Ser Trp Ser Val Pro Cys Gly Pro Cys Ser Glu
               340                 345                 350
   Arg Arg Lys His Leu Phe Val Gln Asp Pro Gln Thr Cys Lys Cys Ser
           355                 360                 365
   Cys Lys Asn Thr Asp Ser Arg Cys Lys Ala Arg Gln Leu Glu Leu Asn
       370                 375                 380
   Glu Arg Thr Cys Arg Cys Asp Lys Pro Arg Arg
   385                 390                 395
<210> 26
   <211> 412
   <212> PRT
   <213> Homo sapiens
<400> 26
   Met Thr Asp Arg Gln Thr Asp Thr Ala Pro Ser Pro Ser Tyr His Leu
   1               5                   10                  15
   Leu Pro Gly Arg Arg Arg Thr Val Asp Ala Ala Ala Ser Arg Gly Gln
               20                  25                  30
   Gly Pro Glu Pro Ala Pro Gly Gly Gly Val Glu Gly Val Gly Ala Arg
           35                  40                  45
   Gly Val Ala Leu Lys Leu Phe Val Gln Leu Leu Gly Cys Ser Arg Phe
       50                  55                  60
   Gly Gly Ala Val Val Arg Ala Gly Glu Ala Glu Pro Ser Gly Ala Ala
   65                  70                  75                  80
   Arg Ser Ala Ser Ser Gly Arg Glu Glu Pro Gln Pro Glu Glu Gly Glu
                   85                  90                  95
   Glu Glu Glu Glu Lys Glu Glu Glu Arg Gly Pro Gln Trp Arg Leu Gly
               100                 105                 110
   Ala Arg Lys Pro Gly Ser Trp Thr Gly Glu Ala Ala Val Cys Ala Asp
           115                 120                 125
   Ser Ala Pro Ala Ala Arg Ala Pro Gln Ala Leu Ala Arg Ala Ser Gly
       130                 135                 140
   Arg Gly Gly Arg Val Ala Arg Arg Gly Ala Glu Glu Ser Gly Pro Pro
   145                 150                 155                 160
   His Ser Pro Ser Arg Arg Gly Ser Ala Ser Arg Ala Gly Pro Gly Arg
                   165                 170                 175
   Ala Ser Glu Thr Met Asn Phe Leu Leu Ser Trp Val His Trp Ser Leu
               180                 185                 190
   Ala Leu Leu Leu Tyr Leu His His Ala Lys Trp Ser Gln Ala Ala Pro
           195                 200                 205
   Met Ala Glu Gly Gly Gly Gln Asn His His Glu Val Val Lys Phe Met
       210                 215                 220
   Asp Val Tyr Gln Arg Ser Tyr Cys His Pro Ile Glu Thr Leu Val Asp
   225                 230                 235                 240
   Ile Phe Gln Glu Tyr Pro Asp Glu Ile Glu Tyr Ile Phe Lys Pro Ser
                   245                 250                 255
   Cys Val Pro Leu Met Arg Cys Gly Gly Cys Cys Asn Asp Glu Gly Leu
               260                 265                 270
   Glu Cys Val Pro Thr Glu Glu Ser Asn Ile Thr Met Gln Ile Met Arg
           275                 280                 285
   Ile Lys Pro His Gln Gly Gln His Ile Gly Glu Met Ser Phe Leu Gln
       290                 295                 300
   His Asn Lys Cys Glu Cys Arg Pro Lys Lys Asp Arg Ala Arg Gln Glu
   305                 310                 315                 320
   Lys Lys Ser Val Arg Gly Lys Gly Lys Gly Gln Lys Arg Lys Arg Lys
                   325                 330                 335
   Lys Ser Arg Tyr Lys Ser Trp Ser Val Tyr Val Gly Ala Arg Cys Cys
               340                 345                 350
   Leu Met Pro Trp Ser Leu Pro Gly Pro His Pro Cys Gly Pro Cys Ser
           355                 360                 365
   Glu Arg Arg Lys His Leu Phe Val Gln Asp Pro Gln Thr Cys Lys Cys
       370                 375                 380
   Ser Cys Lys Asn Thr Asp Ser Arg Cys Lys Ala Arg Gln Leu Glu Leu
   385 390 395 400
   Asn Glu Arg Thr Cys Arg Cys Asp Lys Pro Arg Arg
   405 410
<210> 27
   <211> 371
   <212> PRT
   <213> Homo sapiens
<400> 27
   Met Thr Asp Arg Gln Thr Asp Thr Ala Pro Ser Pro Ser Tyr His Leu
   1               5                   10                  15
   Leu Pro Gly Arg Arg Arg Thr Val Asp Ala Ala Ala Ser Arg Gly Gln
               20                  25                  30
   Gly Pro Glu Pro Ala Pro Gly Gly Gly Val Glu Gly Val Gly Ala Arg
           35                  40                  45
   Gly Val Ala Leu Lys Leu Phe Val Gln Leu Leu Gly Cys Ser Arg Phe
       50                  55                  60
   Gly Gly Ala Val Val Arg Ala Gly Glu Ala Glu Pro Ser Gly Ala Ala
   65                  70                  75                  80
   Arg Ser Ala Ser Ser Gly Arg Glu Glu Pro Gln Pro Glu Glu Gly Glu
                   85                  90                  95
   Glu Glu Glu Glu Lys Glu Glu Glu Arg Gly Pro Gln Trp Arg Leu Gly
               100                 105                 110
   Ala Arg Lys Pro Gly Ser Trp Thr Gly Glu Ala Ala Val Cys Ala Asp
           115                 120                 125
   Ser Ala Pro Ala Ala Arg Ala Pro Gln Ala Leu Ala Arg Ala Ser Gly
       130                 135                 140
   Arg Gly Gly Arg Val Ala Arg Arg Gly Ala Glu Glu Ser Gly Pro Pro
   145                 150                 155                 160
   His Ser Pro Ser Arg Arg Gly Ser Ala Ser Arg Ala Gly Pro Gly Arg
                   165                 170                 175
   Ala Ser Glu Thr Met Asn Phe Leu Leu Ser Trp Val His Trp Ser Leu
               180                 185                 190
   Ala Leu Leu Leu Tyr Leu His His Ala Lys Trp Ser Gln Ala Ala Pro
           195                 200                 205
   Met Ala Glu Gly Gly Gly Gln Asn His His Glu Val Val Lys Phe Met
       210                 215                 220
   Asp Val Tyr Gln Arg Ser Tyr Cys His Pro Ile Glu Thr Leu Val Asp
   225                 230                 235                 240
   Ile Phe Gln Glu Tyr Pro Asp Glu Ile Glu Tyr Ile Phe Lys Pro Ser
                   245                 250                 255
   Cys Val Pro Leu Met Arg Cys Gly Gly Cys Cys Asn Asp Glu Gly Leu
               260                 265                 270
   Glu Cys Val Pro Thr Glu Glu Ser Asn Ile Thr Met Gln Ile Met Arg
           275                 280                 285
   Ile Lys Pro His Gln Gly Gln His Ile Gly Glu Met Ser Phe Leu Gln
       290                 295                 300
   His Asn Lys Cys Glu Cys Arg Pro Lys Lys Asp Arg Ala Arg Gln Glu
   305                 310                 315                 320
   Asn Pro Cys Gly Pro Cys Ser Glu Arg Arg Lys His Leu Phe Val Gln
                   325                 330                 335
   Asp Pro Gln Thr Cys Lys Cys Ser Cys Lys Asn Thr Asp Ser Arg Cys
               340                 345                 350
   Lys Ala Arg Gln Leu Glu Leu Asn Glu Arg Thr Cys Arg Ser Leu Thr
           355                 360                 365
   Arg Lys Asp
       370
<210> 28
   <211> 389
   <212> PRT
   <213> Homo sapiens
<400> 28
   Met Thr Asp Arg Gln Thr Asp Thr Ala Pro Ser Pro Ser Tyr His Leu
   1               5                   10                  15
   Leu Pro Gly Arg Arg Arg Thr Val Asp Ala Ala Ala Ser Arg Gly Gln
               20                  25                  30
   Gly Pro Glu Pro Ala Pro Gly Gly Gly Val Glu Gly Val Gly Ala Arg
           35                  40                  45
   Gly Val Ala Leu Lys Leu Phe Val Gln Leu Leu Gly Cys Ser Arg Phe
       50                  55                  60
   Gly Gly Ala Val Val Arg Ala Gly Glu Ala Glu Pro Ser Gly Ala Ala
   65                  70                  75                  80
   Arg Ser Ala Ser Ser Gly Arg Glu Glu Pro Gln Pro Glu Glu Gly Glu
                   85                  90                  95
   Glu Glu Glu Glu Lys Glu Glu Glu Arg Gly Pro Gln Trp Arg Leu Gly
               100                 105                 110
   Ala Arg Lys Pro Gly Ser Trp Thr Gly Glu Ala Ala Val Cys Ala Asp
           115                 120                 125
   Ser Ala Pro Ala Ala Arg Ala Pro Gln Ala Leu Ala Arg Ala Ser Gly
       130                 135                 140
   Arg Gly Gly Arg Val Ala Arg Arg Gly Ala Glu Glu Ser Gly Pro Pro
   145                 150                 155                 160
   His Ser Pro Ser Arg Arg Gly Ser Ala Ser Arg Ala Gly Pro Gly Arg
                   165                 170                 175
   Ala Ser Glu Thr Met Asn Phe Leu Leu Ser Trp Val His Trp Ser Leu
               180                 185                 190
   Ala Leu Leu Leu Tyr Leu His His Ala Lys Trp Ser Gln Ala Ala Pro
           195                 200                 205
   Met Ala Glu Gly Gly Gly Gln Asn His His Glu Val Val Lys Phe Met
       210                 215                 220
   Asp Val Tyr Gln Arg Ser Tyr Cys His Pro Ile Glu Thr Leu Val Asp
   225                 230                 235                 240
   Ile Phe Gln Glu Tyr Pro Asp Glu Ile Glu Tyr Ile Phe Lys Pro Ser
                   245                 250                 255
   Cys Val Pro Leu Met Arg Cys Gly Gly Cys Cys Asn Asp Glu Gly Leu
               260                 265                 270
   Glu Cys Val Pro Thr Glu Glu Ser Asn Ile Thr Met Gln Ile Met Arg
           275                 280                 285
   Ile Lys Pro His Gln Gly Gln His Ile Gly Glu Met Ser Phe Leu Gln
       290                 295                 300
   His Asn Lys Cys Glu Cys Arg Pro Lys Lys Asp Arg Ala Arg Gln Glu
   305                 310                 315                 320
   Lys Lys Ser Val Arg Gly Lys Gly Lys Gly Gln Lys Arg Lys Arg Lys
                   325                 330                 335
   Lys Ser Arg Pro Cys Gly Pro Cys Ser Glu Arg Arg Lys His Leu Phe
               340                 345                 350
   Val Gln Asp Pro Gln Thr Cys Lys Cys Ser Cys Lys Asn Thr Asp Ser
           355                 360                 365
   Arg Cys Lys Ala Arg Gln Leu Glu Leu Asn Glu Arg Thr Cys Arg Cys
       370                 375                 380
   Asp Lys Pro Arg Arg
   385
<210> 29
   <211> 354
   <212> PRT
   <213> Homo sapiens
<400> 29
   Met Thr Asp Arg Gln Thr Asp Thr Ala Pro Ser Pro Ser Tyr His Leu
   1               5                   10                  15
   Leu Pro Gly Arg Arg Arg Thr Val Asp Ala Ala Ala Ser Arg Gly Gln
               20                  25                  30
   Gly Pro Glu Pro Ala Pro Gly Gly Gly Val Glu Gly Val Gly Ala Arg
           35                  40                  45
   Gly Val Ala Leu Lys Leu Phe Val Gln Leu Leu Gly Cys Ser Arg Phe
       50                  55                  60
   Gly Gly Ala Val Val Arg Ala Gly Glu Ala Glu Pro Ser Gly Ala Ala
   65                  70                  75                  80
   Arg Ser Ala Ser Ser Gly Arg Glu Glu Pro Gln Pro Glu Glu Gly Glu
                   85                  90                  95
   Glu Glu Glu Glu Lys Glu Glu Glu Arg Gly Pro Gln Trp Arg Leu Gly
               100                 105                 110
   Ala Arg Lys Pro Gly Ser Trp Thr Gly Glu Ala Ala Val Cys Ala Asp
   115 120 125
   Ser Ala Pro Ala Ala Arg Ala Pro Gln Ala Leu Ala Arg Ala Ser Gly
   130 135 140
   Arg Gly Gly Arg Val Ala Arg Arg Gly Ala Glu Glu Ser Gly Pro Pro
   145 150 155 160
   His Ser Pro Ser Arg Arg Gly Ser Ala Ser Arg Ala Gly Pro Gly Arg
   165 170 175
   Ala Ser Glu Thr Met Asn Phe Leu Leu Ser Trp Val His Trp Ser Leu
   180 185 190
   Ala Leu Leu Leu Tyr Leu His His Ala Lys Trp Ser Gln Ala Ala Pro
   195 200 205
   Met Ala Glu Gly Gly Gly Gln Asn His His Glu Val Val Lys Phe Met
   210 215 220
   Asp Val Tyr Gln Arg Ser Tyr Cys His Pro Ile Glu Thr Leu Val Asp
   225 230 235 240
   Ile Phe Gln Glu Tyr Pro Asp Glu Ile Glu Tyr Ile Phe Lys Pro Ser
   245 250 255
   Cys Val Pro Leu Met Arg Cys Gly Gly Cys Cys Asn Asp Glu Gly Leu
   260 265 270
   Glu Cys Val Pro Thr Glu Glu Ser Asn Ile Thr Met Gln Ile Met Arg
   275 280 285
   Ile Lys Pro His Gln Gly Gln His Ile Gly Glu Met Ser Phe Leu Gln
   290 295 300
   His Asn Lys Cys Glu Cys Arg Pro Lys Lys Asp Arg Ala Arg Gln Glu
   305 310 315 320
   Asn Pro Cys Gly Pro Cys Ser Glu Arg Arg Lys His Leu Phe Val Gln
   325 330 335
   Asp Pro Gln Thr Cys Lys Cys Ser Cys Lys Asn Thr Asp Ser Arg Cys
   340 345 350
   Lys Met
<210> 30
   <211> 317
   <212> PRT
   <213> Homo sapiens
<400> 30
   Met Thr Asp Arg Gln Thr Asp Thr Ala Pro Ser Pro Ser Tyr His Leu
   1               5                   10                  15
   Leu Pro Gly Arg Arg Arg Thr Val Asp Ala Ala Ala Ser Arg Gly Gln
               20                  25                  30
   Gly Pro Glu Pro Ala Pro Gly Gly Gly Val Glu Gly Val Gly Ala Arg
           35                  40                  45
   Gly Val Ala Leu Lys Leu Phe Val Gln Leu Leu Gly Cys Ser Arg Phe
       50                  55                  60
   Gly Gly Ala Val Val Arg Ala Gly Glu Ala Glu Pro Ser Gly Ala Ala
   65                  70                  75                  80
   Arg Ser Ala Ser Ser Gly Arg Glu Glu Pro Gln Pro Glu Glu Gly Glu
                   85                  90                  95
   Glu Glu Glu Glu Lys Glu Glu Glu Arg Gly Pro Gln Trp Arg Leu Gly
               100                 105                 110
   Ala Arg Lys Pro Gly Ser Trp Thr Gly Glu Ala Ala Val Cys Ala Asp
           115                 120                 125
   Ser Ala Pro Ala Ala Arg Ala Pro Gln Ala Leu Ala Arg Ala Ser Gly
       130                 135                 140
   Arg Gly Gly Arg Val Ala Arg Arg Gly Ala Glu Glu Ser Gly Pro Pro
   145                 150                 155                 160
   His Ser Pro Ser Arg Arg Gly Ser Ala Ser Arg Ala Gly Pro Gly Arg
                   165                 170                 175
   Ala Ser Glu Thr Met Asn Phe Leu Leu Ser Trp Val His Trp Ser Leu
               180                 185                 190
   Ala Leu Leu Leu Tyr Leu His His Ala Lys Trp Ser Gln Ala Ala Pro
           195                 200                 205
   Met Ala Glu Gly Gly Gly Gln Asn His His Glu Val Val Lys Phe Met
       210                 215                 220
   Asp Val Tyr Gln Arg Ser Tyr Cys His Pro Ile Glu Thr Leu Val Asp
   225                 230                 235                 240
   Ile Phe Gln Glu Tyr Pro Asp Glu Ile Glu Tyr Ile Phe Lys Pro Ser
                   245                 250                 255
   Cys Val Pro Leu Met Arg Cys Gly Gly Cys Cys Asn Asp Glu Gly Leu
               260                 265                 270
   Glu Cys Val Pro Thr Glu Glu Ser Asn Ile Thr Met Gln Ile Met Arg
           275                 280                 285
   Ile Lys Pro His Gln Gly Gln His Ile Gly Glu Met Ser Phe Leu Gln
       290                 295                 300
   His Asn Lys Cys Glu Cys Arg Cys Asp Lys Pro Arg Arg
   305                 310                 315

## Description

### FIELD OF THE INVENTION

The invention relates to detection of TB, in particular childhood TB.

### BACKGROUND TO THE INVENTION

Detection of TB is a problem, particularly in children. The current gold standard involves bacteriological assessment. However, sputum samples can be difficult to obtain. Even when successfully obtained, sputum samples from children can exhibit a paucity of bacilli, making direct detection in the sample difficult or impossible. In these circumstances, culture must be carried out in order for the low number of bacilli in the sample to expand to detectable levels. This is laborious and costly and requires specialised laboratory resources, which are drawbacks. More importantly, culture still lacks sensitivity in children. Moreover, culture takes approximately six weeks and this introduces a clinically significant delay to the obtaining of the diagnosis which is a serious problem for patient outcomes. In addition, it is particularly difficult to obtain sputum samples from children, both in practice and in volume. Placing patients, especially children, on speculative treatment without a definitive diagnosisisaserious cost burden as well as the medical risks and complications which such a step would entail.

Dhanasekaran et al 2013 (Genes and Immunity vol 14 pages 356-364) describe identification of biomarkers for *Mycobacterium, tuberculosis* (*M.tb*.) infection and disease in BCG-vaccinated young children in Southern India. A combination of 11 biomarkers isdescribed with only moderate discriminatory power. Unstimulated whole blood supernatants did not identify cytokine expression differences (pages 359-360). Togun et al 2016 (The European Respiratory Journal vol 47 nr 1 pages 223-232) discloses a method for diagnosing TB based on several variables and biomarkers.

WO2014/020343 (Proteinlogic Ltd) discloses biomarkers for diagnosing and/or monitoring tuberculosis. This document is focussed on adults. Childhood TB is mentioned only once, and the age of the subjects is not specified. The only exemplification is confined to adults.

Kumar et al 2013 (Clinical and Vaccine Immunology vol 20 pages 704-711) discloses circulating biomarkers of pulmonary and extrapulmonary tuberculosis in children. It is disclosed that paediatric TB was associated with elevated plasma TGF-beta, IL-21 and IL-23 levels. It is disclosed that no significant differences were found for cytokines, for most type 17 and type 1 interferons, or most cytokines associated with immune modulation.

Hur et al 2015 (Journal of Infection vol 70 pages 346-355) disclose adjunctive biomarkersfor improving diagnosis of tuberculosis and monitoring therapeutic effects. VEGF isdiscussed. No disclosure of childhood TB.

Serene et al 2012 (Biomarkers vol 17 pages 1 - 8) disclose host biomarkers of clinical relevance in tuberculosis: review of gene and protein expression studies. IL-6, IL-22 and IP-10 were mentioned. No disclosure of childhood TB.

Sutherland et al 2012 (PLoS ONE vol 7 epub number: e30324) disclose highly accurate diagnosis of pleural tuberculosis by immunological analysis of the pleural effusion. IL-6 and IP-10 are mentioned. The work is focussed on adults. The work is focussed on pleural fluid.

WO2015/040377 (Medical Research Council) discloses biomarkers for tuberculosis. IL-1ra, FGF and VEGF are mentioned. The work is focussed on adults. The work is focussed on sputum.

The current gold standard for diagnosis is direct detection of the infectious pathogen *Mycobacterium tuberculosis* (*M.tb*.) in a clinical specimen such as sputum.

Differentiating activetuberculosis (TB) disease from other respiratory tract infections (OD) constitutes a major challenge in the management of children with suspected intrathoracic TB disease.

The present invention seeks to overcome problem(s) associated with the prior art.

### SUMMARY OF THE INVENTION

Prior art methods have been based on analysis of sputum, or have been based on extraction and manipulation (such as stimulation) of white blood cells. By contrast, the present inventors have based their detection on indicators which can be found directly in samples taken from the patient. In particular, the inventors have based their detection on a blood sample, and direct detection of markers present in that blood sample. This has the advantage of lending itself to development of a point of care test. This has the advantage of avoiding manipulations and stimulations which are part of the prior art techniques. In particular, the invention is advantageously based on unstimulated blood supernatant.

Thus in oneaspect the invention provides a method for the diagnosis of TB in a subject, the method comprising;
(a) providing a sample from said subject, said sample being selected from the group consisting of: blood, serum and plasma;
(b) determining the concentration in said sample of the following biomarkers: IL-1ra, IL6, IL-7, IL-8, IL-12p70, FGF-basic, IP-10, and VEGF;
(c) converting each biomarker concentration determined in (b) into adecilevalue; and
(d) converting each decile value into a binary presence or absence by comparing the decile values of (c) to the following specific quantile cut-off values:

| **Biomarker** | **Specific quantile cut-off value** |
|---|---|
| IL-1ra | 3 |
| IL6 | 6 |
| IL-7 | 8 |
| IL-8 | 9 |
| IL-12p70 | 9 |
| FGF-basic | 3 |
| IP-10 | 4 |
| VEGF | 9 |

wherein a decile value matching or exceeding the specific quantile cut-off value is converted into the binary presence of the biomarker, and a decile value lower than the specific quantile cut-off valueis converted into the binary absence of the biomarker;
wherein detecting the presence of each of said biomarkers indicates that the subject has TB.

Suitably step (c) converting each concentration determined in (b) into adecilevalue comprises the steps of:
(ci) comparing the concentration of each biomarker determined in (b) to a reference frequency distribution of concentrations of said biomarker; and
(cii) reading out the decile value from the frequency distribution for the concentration of said biomarker.

Suitably step (c) converting each concentration determined in (b) into a decile value comprises the steps of:
(ci) comparing the concentration of each biomarker determined in (b) to a kernel density estimate of concentrations of said biomarker; and
(cii) reading out the decile value from the kernel density estimate for the concentration of said biomarker.

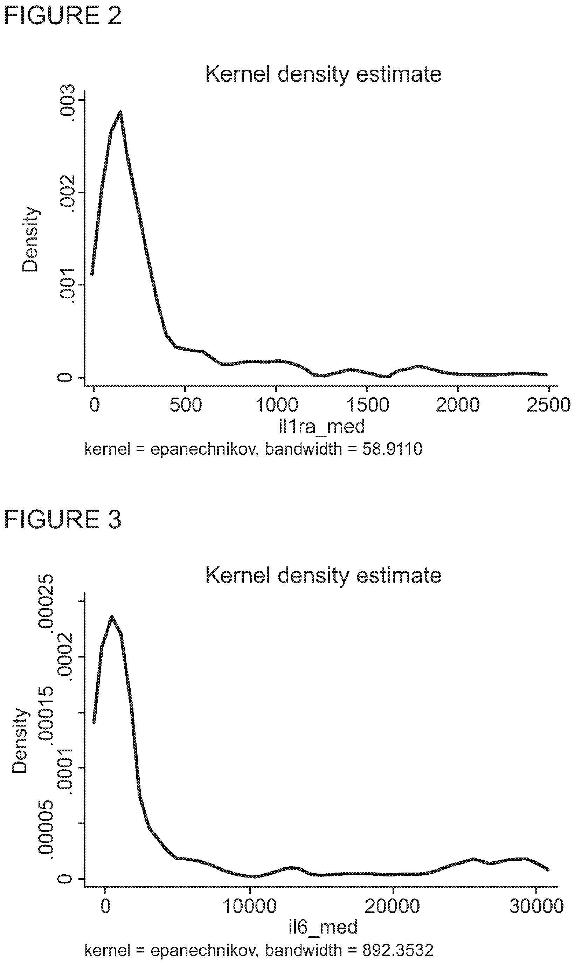

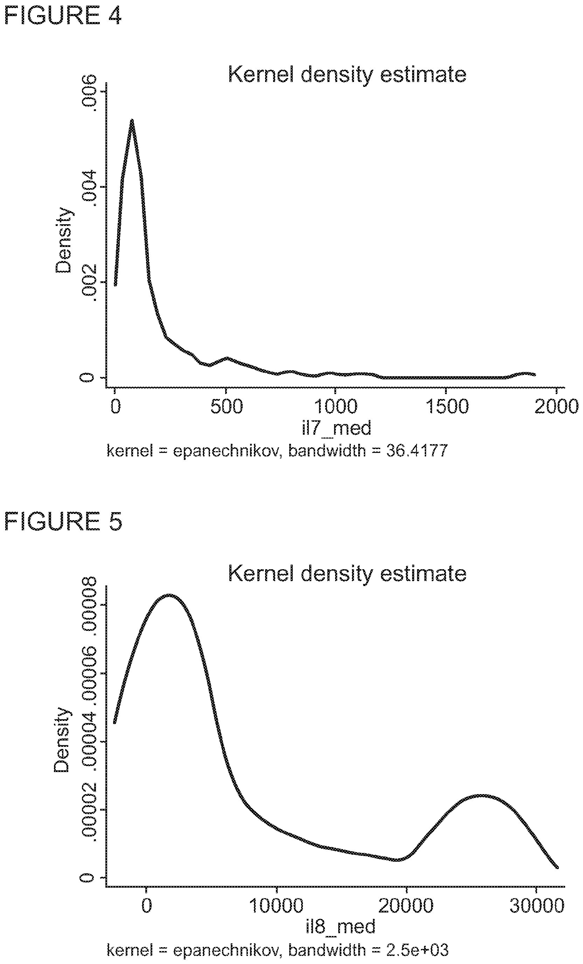

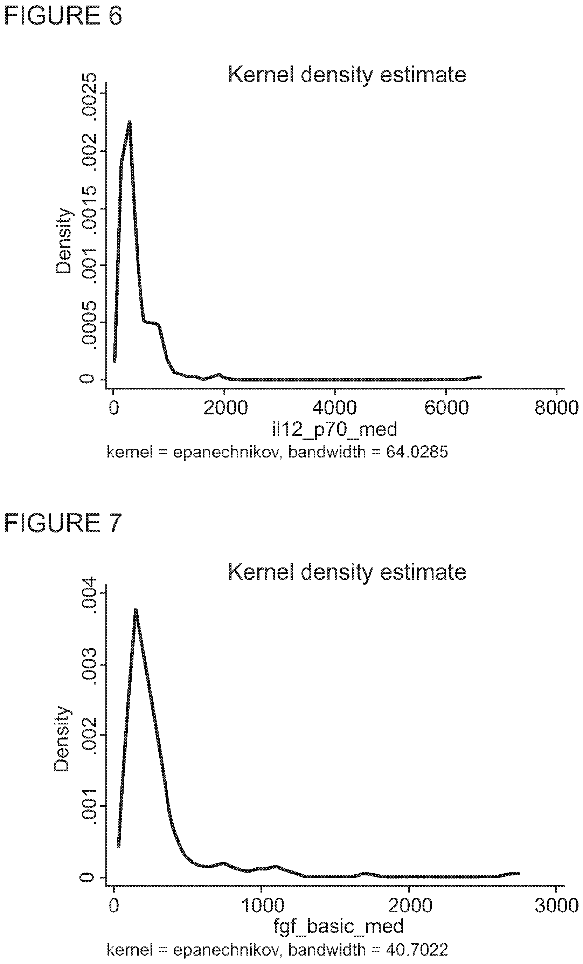

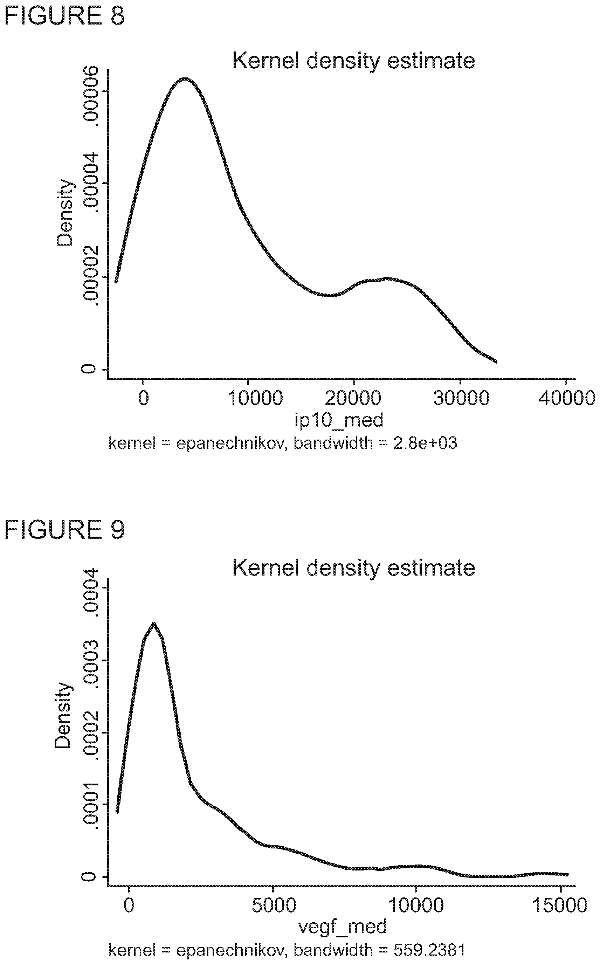

Suitably the reference frequency distribution or kernel density estimate isgenerated by measuring the concentration of the biomarker in a number of subjects, for example a minimum of 100 subjects, and compiling those measurements into a frequency distribution/kernel density estimate. Alternatively the frequency distributions (kernel density estimates) presented in Figures 2 to 9 herein may be used. In this embodiment step (c) converting each concentration determined in (b) into a decile value comprises the steps of:
(ci) comparing the concentration of each biomarker determined in (b) to the corresponding reference frequency distribution / kernel density estimate of concentrations of said biomarker selected from Figures 2 to 9; and
(cii) reading out the decile value from the frequency distribution / kernel density estimate for the concentration of said biomarker.

Suitably determining the concentration of each biomarker comprises:
(bi) detection by contacting the sample with an antibody or antigen binding fragment thereof capable of specifically binding the biomarker; and
(bii) quantification of said binding.

Suitably determining the concentration of each biomarker comprises detection of the mRNA for the biomarker, wherein detection of the mRNA comprises:
(bi) contacting the sample with specific nucleic acid probe(s) or primer(s) for the biomarker; and
(bii) quantification of said probe(s) or primer(s).

Suitably said probe or primer is a non-naturally occurring nucleic acid sequence. Suitably said probe or primer is an artificial or man-made molecule. Suitably said probe or primer is isolated and/or purified. Suitably said probe or primer comprises single stranded nucleic acid. Suitably said probe or primer comprises a label moiety attached thereto. Suitably said label is covalently attached. Suitably said label may be a fluorescent or radioactive label or a Qdot, nanocrystal or nanoparticle, most suitably a fluorescent label.

Suitably said sample is a sample of serum or plasma.

Suitably said serum or plasma is essentially cell free.

Suitably the subject is 16 years old or younger, preferably 15 years old or younger.

Suitably the subject is 2 years old or older, preferably 5 years old or older. Suitably the subject is 5 to 15 years old.

In one embodiment, suitably said method further comprises determining the concentration in said sample of biomarker EC-stimulated VEGF (specific quantile cut-off value 2).

Disclosed is a kit comprising reagent(s) for the specific detection of each of the following biomarkers: IL-1ra, IL6, IL-7, IL-8, IL-12p70, FGF-basic, I P-10, and VEGF.

Further disclosed is a kit comprising reagents for the specific detection of mRNA encoding each of the following biomarkers: IL-1ra, IL6, IL-7, IL-8, IL-12p70, FGF-basic, IP-10, and VEGF.

Suitably said reagents each comprise an antibody or antigen binding fragment thereof selected from the group consisting of a Fab fragment, a Fab' fragment, a F(ab')2 fragment, a scFv, a Fv, a rIgG, and a diabody.

Disclosed is a device comprising an array of materials which together are capable of specifically binding each of the following biomarkers: I L-1ra, IL6, IL-7, IL-8, IL-12p70, FGF-basic, IP-10, and VEGF, each material within the array being capable of specifically binding one of said biomarkers.

Further disclosed is a device comprising an array of materials which together are capable of detecting mRNA specific for each of the following biomarkers: IL-1ra, IL6, IL-7, IL-8, IL-12p70, FGF-basic, IP-10, and VEGF, each material within the array being capable of specifically detecting one of said mRNAs.

Suitably said device is a lateral flow device.

Disclosed is a method of treating a subject comprising carrying out the method as described above, wherein if it is determined that the subject has TB, a regimen of 2HRZE/4HR (2 months HRZE followed by 4 months HR wherein H = isoniazid, R = rifampicin, Z = pyrazinamide, E = ethambutol) is administered to said subject.

In one aspect, the disclosure relates to use of HRZE wherein H = isoniazid, R = rifampicin, Z= pyrazinamide, E = ethambutol for treatment of TB in a subject, wherein the method as described above is carried out for said subject, wherein if it is determined that the subject has TB then HRZE is administered to said subject for two months and then HR is administered to said subject for four months. Suitably a treatment regimen of dosing said subject at least three times per week during the first two months is selected, preferably a treatment regimen of dosing said subject daily during the first two months is selected.

In one aspect, the disclosure relates to tablets of H 75 mg + R 150 mg + Z 400 mg + E 275 mg for treatment of TB in a subject, wherein the method as described above is carried out for said subject, wherein if it is determined that the subject has TB then HRZE is administered to said subject for two months, followed by 3 tablets of H 75 mg + 1.5 tablets of R 150 mg for four months.

In one aspect, the invention relates to a process for selecting a treatment regimen, said process comprising
carrying out the method as described above, wherein if it is determined that the subject has TB then a treatment regimen of 2HRZE/4HR (2 months HRZE followed by 4 months HR wherein H = isoniazid, R = rifampicin, Z = pyrazinamide, E = ethambutol) is selected.

In one aspect, the invention relates to use of a combination of materials each of which recognises, specifically binds to or has affinity for one of the following biomarkers: IL-1ra, IL6, IL-7, IL-8, IL-12p70, FGF-basic, IP-10, and VEGF, wherein said combination includes at least one such material for each of said biomarkers, for aiding diagnosis of TB in a subject. Suitably said material comprises an antibody or antigen binding fragment thereof.

In one aspect, the disclosure relates to use for aiding diagnosis of TB in a subject, of a combination of materials each of which recognises, specifically binds to or has affinity for mRNA of one or more of the following biomarkers: IL-1ra, IL6, IL-7, IL-8, IL-12p70, FGF-basic, IP-10, and VEGF. Suitably said material comprises a nucleic acid primer or probe.

In one aspect, the invention relates to a apparatus comprising logic configured to carry out the method as described above.

In one aspect, the invention relates to a computer program product operable, when executed on a computer, to perform the method steps as described above.

### DETAILED DESCRIPTION OF THE INVENTION

The inventors teach identification of novel host biomarkers for childhood TB. The inventors hypothesised that a unique biosignature for TB disease in children could be identified.

'TB' means Tuberculosis; this is a disease caused by the bacterium *Mycobacterium tuberculosis* (sometimes referred to asMTB).

It has been a challenge in the field of TB to provide a simple diagnostic test. The test needs to be reliable. The test needs to be accurate. The test should ideally involve the minimum of tooling/equipment, especially since TB is often a problem in developing countries where laboratory facilities can be few and/or can be geographically distant from the patients being tested.

### SAMPLE

The sample may be from a subject. The subject is suitably a mammal, most suitably a human.

Suitably the methods do not involve actual collection of the sample. Suitably the sample is an *in vitro* sample.

Methods of the invention are suitably performed on an isolated sample from the subject being investigated. Thus, suitably the methods are methods which may be conducted in a laboratory setting without the need for the subject to be present. Suitably the methods are carried out *in vitro* i.e. suitably the methods are *in vitro* methods. Suitably the methods are extracorporeal methods.

Suitably the invention may be applied to analysis of nucleic acids. Suitably, nucleic acid is prepared from the sample collected from the subject of interest, e.g. by extraction of nucleic acid from white blood cells in the sample. Suitably, the sample comprises nucleic acid. Suitably, the sample consists of nucleic acid. Suitably, the nucleic acid comprises, or is, mRNA or cDNA, suitably mRNA.

Most suitably the invention may be applied to analysis of protein biomarkers. Most suitably proteins in the sample are analysed.

Suitably the sample is an *in vitro* sample.

Suitably the sample is an extracorporeal sample.

Suitably the sample is blood.

Suitably the sample is blood supernatant.

Suitably the sample is serum. Serum may be obtained as the fluid collected from a blood sample which has been clotted.

Suitably the sample is plasma. Plasma may be obtained as the fluid collected from a blood sample which has been centrifuged to pellet the blood cells present. Alternatively plasma may be obtained by filtration to remove the blood cells present.

Suitably the blood or blood supernatant/serum/ plasma is unstimulated.

It is an advantage of the invention that the analysis converts each measured biomarker into a binary output. For example, each cytokine biomarker examined is turned into a binary YES/NO data point. By contrast, existing prior art approaches need quantitative readouts.

It is an advantage of the invention that diagnostic accuracy is achieved at a level comparable to bacteriological culture. It is an advantage of the invention that there are no time delays comparable to those experienced with bacteriological culture.

Prior art techniques use cells, either bacterial cells or isolated blood cells. It is an advantage of the invention that no cells are required to be cultured, and no cells are required to be isolated.

It is an advantage that the host response/host signature is analysed. Without wishing to be bound by theory, the existing attempts to create a blood test for TB have focussed on challenging or stimulating immune cells from the subject and studying the response. However, in principle this approach is always examining a "recall response". This is a response derived from "memory" of the immune system which has previously encountered a TB bacterium. Firstly, this requires a stimulation of the sample either with bacteria or with antigens, which is labour intensive and costly. However, more importantly, in principle, this type of approach is only able to assay a secondary response. By contrast, the present disclosure is concerned with assessment of the primary response. This is especially important and useful when applied to children, since clearly each individual patient is at some point in their lives exposed to TB for the first time. If a method such asaprior art method is only focussed on assessing a secondary response, then it is unlikely ever to be able to detect a response the first time such a subject encounters TB. It is an advantage that the direct primary response is being assessed.

Suitably the sample is a cell free sample. Suitably the sample does not comprise cells. Suitably the cells are removed from the blood sample by centrifugation and retrieval of the supernatant.

Cells may be removed from the sample by any method known in the art. For example filtration.

A key reason for excluding cellsfrom the analysis is because blood is deeply red in colour due to the presence of the erythrocyte red blood cells. Typically the detection step used to assess the presence or absence of the biomarkers is light sensitive. Therefore, advantageously the sample is free of cells in order to avoid being confounded by the red colour of whole blood. In principle, any approach which circumvents the deep red colour of whole blood could be employed. Suitably this may be by cell lysis. More suitably the cells are removed from the blood before analysis. Suitably this is by centrifugation. Suitably this may be by filtration. Suitably this may be by lateral flow.

In some settings, it may be possible to use awhole blood sample in the method of the invention, for example by using lateral flow. In this scenario, the whole blood sample is placed in a lateral flow device. The fluid component such as plasma/serum then migrates, so at tile point of assessment it is cell free.

### LATERAL FLOW ASSAY AND OTHER DEVICES

Lateral flows test assays (LFA) - also referred to as lateral flow immunochromatographic assays- require minimal infrastructure and have been used to develop cheap and simple devices for rapid medical diagnosis and screening, point of care tests or laboratory use. The assay is based on the detection of the presence of a specified target analyte in a sample for mostly qualitative and occasional quantitative analysis. Common applications of the LFA include its use in home pregnancy test, monitoring of diabetes and rapid diagnosis of HIV or parasitic and bacterial infections. As discussed extensively in review articles (such as Sajid M, Kaw de A, Daud M. Designs, formats and applications of lateral flow assay: A literature review. Journal of Saudi Chemical Society. 2014), a typical LFA strip is made up of four parts:
- **Sample application pad:** This is an absorbent pad made of cellulose or glass fibre on which the sample is applied and its main function is to transport the sample (e.g. blood) containing the analyte 'downstream' to the other part of the LFA strip. It may also pre-treat the sample, including separation into components such as plasma, before its transportation.
- **Conjugate pad:** this contains immobilized and labelled antibody that is specific for the target analyte. The antibody is conjugated to coloured particles such as latex or nanometre sized particles and the labelled antibody conjugate is released upon contact with moving liquid sample.
- **Nitrocellulose or Reaction membrane:** This membrane allows movement of complexes generated from the conjugated pad under capillary action. The membrane is further divided into test and control lines.
- **Adsorbent pad:** This pad works as a 'sink' at the end of the strip and it is designed to further draw the sample across the reaction by capillary action.

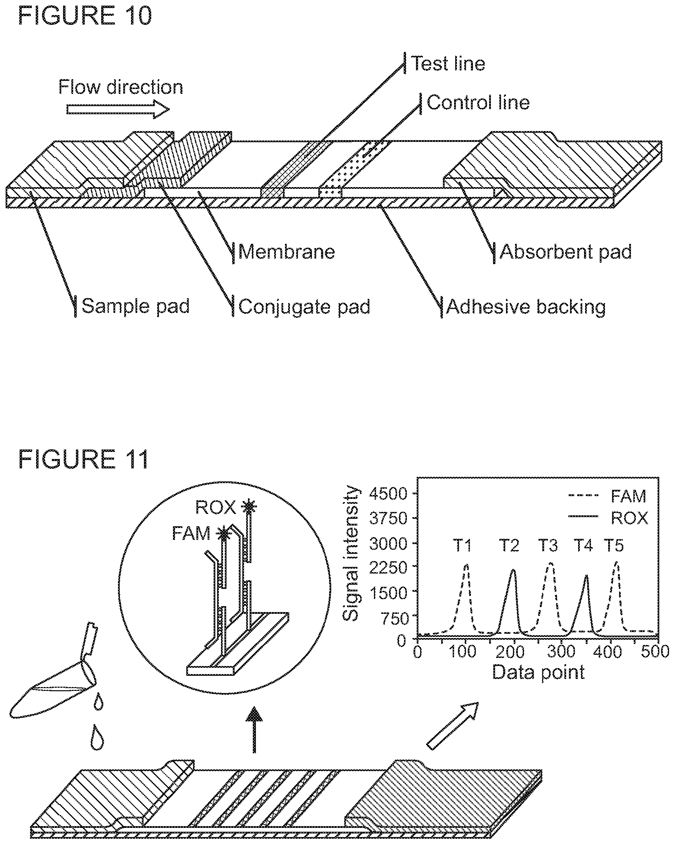

Figure 10 shows the standard layout of a lateral flow device (Saied Assadollahi, Christiane Reininger, Roland Palkovits, Peter Pointl and Thomas Schalkhammer. 'From Lateral Flow Devices to a Novel Nano-Color Microfluidic Assay.' Sensors 2009(9);6084-6100; doi:10.3390/s90806084).

Lateral flow assay test can work in two main formats, which are the sandwich and competitive assays. Sandwich LFA are designed for detection of large molecular weight molecules including proteins with multiple antigenic sites (e.g. HIV, hCG), while competitive assays are designed to test small molecules with single epitopes. In sandwich LFA, a positive test will show coloured bands on the test line, while in competitive LFA the test line will show coloured band in negative samples.

### Multiplex detection format

In clinical diagnosis, the higher specificity or predictive accuracy of multiple interdependent analytes that are detected simultaneously under the same condition has led to the development of multiplex LFA detection format used for the detection of more than one target analyte(Panhotra BR, Hassan ZU, Joshi CS, Bahrani A. Visual detection of multiple viral amplicons by dipstick assay: its application in screening of blood donors a welcome tool for the limited resource settings. Journal of clinical microbiology. 2005 Dec;43(12):6218; author reply-9. PubMed PMID: 16333138. Pubmed Central PMCID: 1317223; Corstjens PL, de Dood CJ, van der Ploeg-van Schip JJ, Wiesmeijer KC, Riuttamaki T, van Meijgaarden KE, et al. Lateral flow assay for simultaneous detection of cellular- and humoral immune responses. Clinical biochemistry. 2011 Oct;44(14-15):1241-6. PubMed PMID: 21763300. Pubmed Central PMCID: 3177995). In this format, the assay is performed over a strip with number of test lines equal to the number of target analytes as recently described for the detection of four common human papillomavirus (HPV) types (Xu Y, Liu Y, Wu Y, XiaX, Liao Y, Li Q. Fluorescent probe-based lateral flow assay for multiplex nucleic acid detection. Analytical chemistry. 2014 Jun 17;86(12):5611-4. PubMed PMID: 24892496).

Figure 11 shows multiplex detection format of LFA (Ye Xu, Yinghua Liu, Yan Wu, Xiaohu Xia, Yiqun Liao and Qingge Li. 'Flourescent Probe-Based Lateral Flow Assay for multiplex Nucleic Acid Detection.' Anal Chem., 2014, 86(12), 5611-5614).

### Clinical applications

Lateral Flow Assays have found important use in clinical diagnosis and as point of care tests through the detection of clinical analytes in plasma, serum, urine and other clinical samples. A ready exampleisthe home pregnancy testing kits. In tuberculosis (TB), the urinary lipoarabinomannan (LAM) test is a LFA test with low sensitivity (52 - 59% but a high specificity (>94%) (Lawn SD, Dheda K, Kerkhoff AD, Peter JG, Dorman S, Boehme CC, et al. Determine TB-LAM lateral flow urine antigen assay for HIV-associated tuberculosis: recommendations on the design and reporting of clinical studies. BMC infectious diseases. 2013;13:407. PubMed PMID: 24004840. Pubmed Central PMCID: 3846798). The inventors also recently reported that the use of fluorescent up-converting phosphor (UCP) reporter technology combined with LFA to detect IP-10 and CCL4 simultaneously on the same strip has potential to be developed as a point of care test for pleural TB (Sutherland JS, Mendy JF, Gindeh A, Walzl G, Togun T, Owolabi O, et al. Use of lateral flow assays to determine IP-10 and CCL4 levels in pleural effusions and whole blood for TB diagnosis. Tuberculosis (Edinb). 2015).

A multi-centre study in Africa similarly reported the applicability of the low-tech and robust UCP-LFA platform as a convenient quantitative assay for detection of multiple chemokines in whole blood (Corstjens PL, Tjon Kon Fat EM, de Dood CJ, van der Ploeg-van Schip JJ, Franken KL, Chegou NN, et al. Multi-center evaluation of a user-friendly lateral flow assay to determine IP-10 and CCL4 levels in blood of TB and non-TB cases in Africa. Clinical biochemistry. 2015 Aug 15. PubMed PMID: 26285074).

Therefore, the disclosure relates to a device comprising an array of materials which together are capable of binding each of the following biomarkers: IL-1ra, IL6, IL-7, IL-8, IL-12p70, FGF-basic, IP-10, and VEGF, each material within the array being capable of binding one of said biomarkers, wherein said device is a lateral flow device. In this embodiment, the array of materials suitably comprises a number of test lines equal to the number of biomarkers. Suitably each test line comprises material capable of binding one such biomarker. Suitably each test line comprises material capable of binding a different such biomarker.

Alternatively, if the device comprises a 'chip' or 'biochip', the array may comprise a spatial arrangement of the materials such as a grid or other defined arrangement such as a geometric pattern.

Suitably each material capable of binding one of said biomarkers is immobilised within the device.

Suitably each material capable of binding one of said biomarkers is modified.

Suitably each material capable of binding one of said biomarkers is labelled. Suitably the label is covalently attached. Suitably the label is a dye.

Suitably each material capable of binding one of said biomarkersis adifferent antibody or antigen binding fragment thereof, wherein the antigen binding fragment thereof is selected from the group consisting of a Fab fragment, a Fab' fragment, a F(ab')2 fragment, a scFv, a Fv, a rlgG, and a diabody.

Suitably the antibody or antigen binding fragment thereof is a non-human antibody or antigen binding fragment thereof. Suitably the antibody or antigen binding fragment thereof is recombinant, e.g. made by *in vitro* expression of a recombinant nucleic acid sequence. Suitably the antibody or antigen binding fragment thereof is purified and/or isolated.

Non-antibody reagents for detection e.g. of protein biomarkers as described above may also be used, (e.g. for detection in the methods, as material in the devices, as reagents in the kits, or other applications of the invention) for example phagedisplay particles offering specific binding peptides, affimers, aptamers, nucleic acids binding specific protein or peptide sequences, small molecules with specific binding properties or other such specific binding partner(s) of the biomarkers described.

### SIGNATURE

Prior art approaches have identified extremely large signatures such as requiring the analysis of 50 or more individual biomarkers. It is an advantage of the invention that the signature requires analysis of only 8 biomarkers.

Prior art attempts have involved flow cytometry. However, flow cytometry is extremely labour intensive, expensive, and is unsuitable for the provision of a bedside/point of care test.

Prior art approaches have involved analysis of the transcriptome (i.e. of mRNAs). However, these approaches have typically also involved assessment of at least 50 genes, which is a problem.

Data is presented in the examples section in support of the AUC/specificity/sensitivity of the methods of the invention.

Most importantly, the positive and negative predictive values are provided. In the field of TB, the predictive values may be regarded as more important even than the sensitivity/ specificity of the method. It is an advantage that the invention delivers extremely robust positive and negative predictive values. This is discussed in more detail in the section below entitled "Predictive Values/ Applications Of The Method"

The inventors undertook a large and complex analysis involving numerous intellectual choices in arriving at the 8 biomarker signature. In particular, it is important to note that this particular signature has special properties. For example, it is significantly different from a 7 biomarkers signature, which is unsuitable. Analysis of the signature attempting to drop any of the8 biomarkers disclosed leads to adrop in specificity of approximately 50% and/or adrop in predictive value of approximately 25%. These figures clearly illustrate that the teaching of an 8 biomarker signature according to the invention is not merely an iterative or arbitrary choice but presents clinically useful information which is a step change from that obtained with even one fewer marker. It is surprising that such a sharp and dramatic effect can be observed in this manner.

Suitably the signature comprises 8 biomarkers.

Suitably the biomarkers are as set out in the table below.

### REFERENCE SEQUENCES

Suitably the reference sequences of the biomarkers of interest are as defined in the following table:

| **Biomarker** | **Protein Accession Number (UniProtKB)** | **Canonical sequence** | **Protein Isoforms** | **Gene name** | **mRNA Accession Number (RefSeq Release 73)** |
|---|---|---|---|---|---|
| IL-1ra | P18510 | P18510-1 | P18510-2 | *IL1RN* | NM_173841.2 |
| | | | P18510-3 | | NM_000577.4 |
| | | | P18510-4 | | NM_173842.2 |
| | | | | | NM_173843.2 |
| IL6 | P05231 | P05231-1 | | *IL6* | NM_000600.3 |
| IL-7 | P13232 | P13232-1 | P13232-2 | *IL7* | NM_000880.1 |
| | | | P13232-3 | | NM_001199886.1 |
| | | | | | NM_001199887.1 |
| | | | | | NM_001199888.1 |
| IL-8 | P10145 | P10145-1 | P10145-2 | *CXCL8* | NM_000584.3 |
| IL-12p70 | P29460 | P29460-1 | | *IL12B* | NM_002187.2 |
| FGF-basic | P09038 | P09038-4 | P09038-1 | *FGF2* | NM_002006.4 |
| | | | P09038-2 | | |
| | | | P09038-3 | | |
| IP-10 | P02778 | P02778-1 | | *CXCL10* | NM_001565.3 |
| VEGF | P15692 | P15692-1 | P15692-2 | *VEGFA* | NM_001025366.2 |
| | | | P15692-3 | | NM_001025367.2 |
| | | | P15692-4 | | NM_001025368.2 |
| | | | P15692-5 | | NM_001025369.2 |
| | | | P15692-6 | | NM_001025370.2 |
| | | | P15692-8 | | NM_001033756.2 |
| | | | P15692-9 | | NM_001171622.1 |
| | | | P15692-10 | | NM_001171623.1 |
| | | | P15692-11 | | NM_001171624.1 |
| | | | P15692-12 | | NM_001171625.1 |
| | | | P15692-13 | | NM_001171626.1 |
| | | | P15692-14 | | NM_001171627.1 |
| | | | P15692-15 | | NM_001171628.1 |
| | | | P15692-16 | | NM_001171629.1 |
| | | | P15692-17 | | NM_001171630.1 |
| | | | P15692-18 | | NM_001204384.1 |
| | | | | | NM_001204385.1 |
| | | | | | NM_001287044.1 |
| | | | | | NM_001317010.1 |
| | | | | | NM_003376.5 |

The sequences are hereby incorporated herein by reference.

The skilled worker only has to identify the correct gene/ protein being used in the analysis. The guidance provided is not intended to restrict the invention rigidly to the specific single exemplary sequences provided. Gene sequences (and therefore protein sequences) are known to vary between individuals e.g. due to allelic variance or mutations between individuals. The information provided is to assist the operator in working the invention by assaying the correct gene. Ultimately the gene product (such as mRNA or more suitably protein) is actually assayed. Therefore minor or minimal allelic or mutational differences between individuals are not important, what is important is that the correct gene (gene product) is assayed using the guidance provided.

Suitably where the biomarker name is used, this means the corresponding amino acid or nucleic acid sequence from the above table. Suitably for amino acid sequences, the canonical sequence is preferred. Suitably for nucleic acid sequences, the most recent (e.g. highest numbered) nucleic acid sequence is preferred.

It will be understood that the invention may equally make use of detection of fragment(s), variant(s) or mutant(s) of these biomarkers. Suitably any such fragment(s), variant(s) or mutant(s) have at least 80% sequence identity to the reference sequences along the whole length of said fragment(s), variant(s) or mutant(s), suitably 90%, suitably 95%, suitably 98% sequence identity along the whole length of said fragment(s), variant(s) or mutant(s).

### DATABASE RELEASE

Sequences deposited in databases can change over time. Suitably the current version of sequence database(s) are relied upon. Alternatively, the release in force at the date of filing is relied upon.

As the skilled person knows, the accession numbers may be version/dated accession numbers. The citeable accession numbers for the current database entry are the same as above, but omitting the decimal point and any subsequent digits e.g. for VEGF a version/dated accession number is P15692-18; the current entry is obtained using P15692 and so on.

GenBank is the NIH genetic sequence database, an annotated collection of all publicly available DNA sequences (National Center for Biotechnology Information, U.S. National Library of Medicine 8600 Rockville Pike, Bethesda MD, 20894 USA; Nucleic Acids Research, 2013 Jan;41(D1):D36-42) and accession numbers provided relate to this unless otherwise apparent. Suitably the GenBank database release referred to is 15 October 2015, NCBI-GenBank Release 210.0.

UniProt (Universal Protein Resource) is a comprehensive catalogue of information on proteins ('UniProt: a hub for protein information' Nucleic Acids Res. 43: D204-D212 (2015).). For the avoidance of doubt, UniProt Release 2015_11 is relied upon.

In more detail , the UniProt consortium European Bioinformatics Institute (EBI), SIB Swiss Institute of Bioinformatics and Protein Information Resource (PIR)'s UniProt Knowledgebase(UniProtKB) Release 2015_11 (11-Nov-2015) is relied upon.

### TREATMENTS

It should be noted that treatment for TB is a minimum six month program of drugs. This is expensive and can be very demanding on the patient. Dosage has to be very regular, such as multiple doses per week and ideally daily, which is a heavy burden on the healthcare provider as well as the patient. Therefore, it is a problem in the art to avoid the mistreatment of patients i.e. the mis-prescription of TB drugs to a patient who does not in fact have TB. The present invention alleviates this problem by providing a robust tool for diagnosis (or for aiding diagnosis).

If it isdecided in view of the method of the invention that the subject has TB, then the physician should prescribe the treatment for TB. Provided is a method of treating a patient comprising determining if they have TB according to the method(s) disclosed herein, wherein if the patient is determined to have TB then the treatment for TB is prescribed, or more suitably administered. In another embodiment the methods of the invention are used to aid diagnosis of a patient who need not be present during the diagnostic step in the strict sense (i.e. in this embodiment the invention is not directed at diagnosis for curative purposes *stricto sensu*); the physician may then take the information provided by the invention into account when diagnosing and/or planning the treatment for said subject.

Thus it is an advantage of the invention that unnecessary drugs can be avoided. In one embodiment, the test of the invention finds application as a rule-in test rather than a rule-out test. In other words, if the method(s) of the invention are used to determine that a subject has TB, they should definitely be treated for TB. In the alternative, if the methods of the invention are used and it is not determined that a subject hasTB, then further investigation may be useful - asubject is suitably not 'ruled out' from possibly having TB if the methods of the invention do not determine that they have TB.

In terms of a'rule-in' test, the methods of the invention have a comparable performance to the gold standard in the art (bacterial culture). Thus it isan advantage of the invention that a positive finding using the methods of the invention provides a very high level of confidence that the subject has TB and should be treated for TB.

Suitably TB treatment is as recommended by the World Health Organisation (WHO). The WHO may amend its guidelines from time to time - suitably treatment is as per the guidelines at the date of working the invention to treat a subject. More suitably this treatment is as per the guidelines at the filing date of this document. If any further guidance is needed, most suitably treatment is as per the guidelines in the WHO 2010 publication "Guidelines for national programmes, fourth edition" ISBN: 9789241547833 (e.g. http://www.who.int/tb/publications/9789241547833/en/). This document is hereby incorporated herein by reference, specifically for the teaching of the specific treatment regime for TB.

Exemplary treatments are set out below.

Suitably TB treatment comprises the standard 6-month course of 4 antimicrobial drugs as recommended by the WHO.

Suitably TB treatment comprises 6 months of rifampicin.

Suitably TB treatment comprises six (6) months treatment with specific anti-TB drugs (Isoniazid, Rifampicin, Pyrazinamide amd Ethambutol for first 2 months, followed by 4 months of Isoniazid and Rifampicin only).

Suitably patients with TB may receive a daily intensive phase followed by a three times weekly continuation phase [2HRZE/ 4(HR)3] ; suitably each dose is directly observed. Three times weekly dosing throughout therapy [2(HRZE)3/4(HR)3] may be used as an alternative, provided that every dose is directly observed and the patient is NOT living with HIV or living in an HIV-prevalent setting.

Suitably the dosing is not less than 3 times per week. Suitably the dosing is 3 times per week or more. Most suitably the dosing is daily.

Most suitably the dosing frequency for patients with TB isdaily throughout the course of therapy [2HRZE/4HR].

Suitably if the patient is living with HIV or living in an HIV-prevalent setting the dosing is daily throughout the course of therapy.

Summary of WHO treatment guidance (WHO 2010 publication "Guidelines for national programmes, fourth edition" page5):

**Table A STANDARD REGIMEN AND DOSING FREQUENCY FOR NEW TB PATIENTS**

| Intensive phase | Continuation phase | Comments |
|---|---|---|
| 2 months of HRZE^{a} | 4 months of HR | |
| 2 months of HRZE | 4 months of HRE | Applies only in countries with high levels of isoniazid resistance in new TB patients, and where isoniazid drug susceptibility testing in new patients is not done (or results are unavailable) before the continuation phase begins |
| ^{a} WHO no longer recommends omission of ethambutol during the intensive phase of tretment for patients with non-cavitary, smear-negative pulmonary TB or extrapulmonary disease who are known to be HIV-negative. | | |
| | | |

| Dosing frequency | | Comments |
|---|---|---|
| Intensive phase | Continuation phase | |
| Daily | Daily | Optimal |
| Daily | 3 times per week | Acceptable alternative for any new TB patient receiving directly observed therapy |
| 3 times per week | 3 times per week | Acceptable alternative provided that the patient is receiving directly observed therapy and is NOT living with HIV or living in an HIV-prevalent setting (see Chapter 5) |

| | | |
|---|---|---|
| *Note*: Dally (rather than three times weekly) intensive-phase dosing may help to prevent acquired drug resistance in TB patients starting treatment with isoniazid resistance (see Annex 2). | | |

### Abbreviations used:

H = isoniazid,
R = rifampicin,
Z = pyrazinamide,
E = ethambutol,
S = streptomycin.

The standard treatment above is suitably not applied to muti drug resistant TB (MDR TB). Suitably drug susceptibility testing is undertaken before treatment, in accordance with WHO guidelines. If treating a TB patient whose treatment has failed or other patient with high likelihood of multidrug-resistant TB (MDR-TB), suitably treatment should be started on an empirical MDR regimen as recommended by WHO. Most suitably the invention is applied to 'new' TB, i.e. new patients tested according to the invention.

Further or additional treatment may depend on whichever diagnosis is made; usually a course of antibiotics if it is a bacterial respiratory tract infection.

Administration may be by tablet or by injection such as intramuscular injection. For HRZE/HR regimes, suitably administration is by tablet.
Typical tablets comprise the following doses:
H 75 mg + R 150 mg + Z 400 mg + E 275 mg tablets.
Subjects are administered or prescribed a number of tablets according to their body weight (and/or any other relevant factors if necessary) to achieve the correct dose. This number may include fractions of a tablet. A typical dose for a subject of 30-39Kg body weight is 2 tablets of H 75 mg + R 150 mg + Z 400 mg + E 275 mg daily during the first 2 months of treatment, followed by 1.5 tablets of H 150 mg + R 150 mg during months 3 to 6 of treatment. The determination of exact dose e.g. based on body weight is a matter for the physician.

### STATISTICAL ANALYSIS

The inventors considered standard approaches to statistical analysis. However, the inventors had insight that standard regression models tend to lead to over optimism. This is particularly true considering the multidimensionality of the data arising from the large number of cytokine covariates some of which are highly correlated with each other. For various reasons, the inventors used a different approach, and simultaneously tried to shrink the marker set (i.e. to reduce the dimensionality) whilst applying penalties for shrinkage in the statistical analysis. Without wishing to be bound by theory, their reasoning was that if a signature assesses two markers which move in the same direction, then qualitatively the same information is being obtained from two comparable sources. This provides an opportunity to eliminate one of those markers, thereby simplifying the signature without compromising the quality of information obtained. Thecorollary of this is that if two analyses are providing information in two different directions, then this can be seen to provide extra information to improve the signature. In this manner, the inventors sought to remove any markers which might be considered as statistically related to one another, thereby arriving at an improved empirical (reduced) signature but which still delivered excellent diagnostic characteristics.

### MARKER SELECTION

The inventors undertook unbiased marker selection. Prior art based approaches have tended to use a "case control" approach. In brief, this might be characterised by settling on TB as asubject to be addressed, selecting healthy patients, selecting patients having TB, and comparing the healthy patients with the TB diseased patients. By contrast, the inventors designed a case-control study nested within a prospective cohort (i.e. nested case-control) approach. They selected a cohort of children using the same selection process to identify children in different geographical locations. Only then did they identify within those cohorts patients having TB and patients not having TB. More importantly, the inventors chose to compare TB to OD (i.e. "other respiratory disease but not TB"). This is because it is a key clinical decision to identify those patients with TB compared to those presenting with other diseases which are not TB. Thus, it can be considered a problem in the art to differentiate TB patients from "other disease" patients.

Another drawback with prior art studies is that they have tended to settle on biomarkers such as cytokines which have been published or associated with TB. By contrast, the inventors took an entirely unbiased approach and did not pick biomarkers by association with TB. They undertook a blind analysis and arrived at the signature of 8 biomarkers without knowing what those individual biomarkers were. Only then did the inventors analyse the identities of the biomarkers in their signature. One example of the surprise of this approach is by considering IFN-γ. The view in the art is that IFN-γ is involved with TB. Indeed, prior art approaches have tried to use IFN-γ and/ or IP-10 as biomarkers for TB. It is extremely surprising that the biomarker signature taught herein does not comprise IFN-γ.

Quantiferon ('QFT' - available commercially from Qiagen Inc.) isan interferon-gamma (IFN-γ) release assay, commonly known as an IGRA, and is a modern alternative to the tuberculin skin test (TST or Mantoux). In overview, QFT measures the cell-mediated immune response (cytokines) to very specificTB antigens. The test is performed by collecting wholeblood (1 mL) into each of three blood collection tubes. When the blood of an infected patient is stimulated with the *M. tuberculosis*-specific antigens in QFT, their T-Cells respond by secreting acytokine called IFN-y. The IFN-y concentration in the plasma is determined using a sensitive ELISA.

Thus, Quantiferon ('QFT') is a commercial assay that still measures IFN-gamma following stimulation of blood with antigens specific for *M.tb.* Some IP-10 assays have also been investigated in the prior art. However, while IP-10 is reportedly more robust than IFN-gamma (i.e. released at a much higher level following stimulation), on its own it cannot distinguish between TB disease and TB sensitization similar to I FN-gamma, which is a problem in the art. Advantageously, in the present invention, what is taught is a combination of markers, which when used together - not individually - can distinguish TB from OD. This is based on the inventors' insight that, given the complexity of TB disease, a combination of markers rather than a single marker has a higher power and specificity to distinguish TB from TB sensitization and/or other disease.

In more detail, the inventors took an unusual approach by analysing various cytokines and chemokines and turning the values of each into deciles. Starting with 27 cytokines/chemokines as candidates, each was transformed into 10 deciles giving 270 for each patient stimulated and 270 for each patient unstimulated.

This represents a categorical (rather than continuous) approach which is itself a key part of the innovative approach taken. This approach has never before been applied to cytokine analysis. This has advantages which include removing the confounding effect of bias or selection in the biomarkers which are used.

The panel of 27 initial candidates did not have any previous association with TB. For example, they were not TB specific. At most, they may be regarded as an "immune panel". They are simply markers involved in lymphocytes as part of a commercial kit which is not in any way marketed or directed towards TB. To illustrate how surprising the findings were, even the inventors did not predict what they would find by using this approach.

In one embodiment the analysis may be carried out as follows:
frequency distribution >10 deciles>makee ach a binary quantile.

In one embodiment the analysis may be carried out as follows:
frequency distribution > deciles>10 - equal sized quantiles> use each quantile as cut-off to generate a binary variable.

It should be noted that each cytokine may be present across a different range of concentrations so that each of the deciles may not be the same between individual biomarkers. However, the distribution of each individual biomarker is suitably divided into 10 deciles (i.e. 10 equal-sized quantiles) according to its own range of concentrations when present; each biomarker value determined for a patient is then converted into a decile from that frequency distribution.

It should be noted that the invention is directed at obtaining a clinical decision whether a subject has TB or OD.

It is possible to use the invention as a screening tool such as a population screening test.

It is an advantage of the invention that it is helpful in assisting the clinical decision whether a patient has TB or OD.

### DECILE DETERMINATION

Deciles are generated according to standard statistical techniques. Generation of deciles is a mathematical frequentist procedure that can be derived or generated by any statistical software. A specific variable (e.g. a measured cytokine) with quantitative values when measured from several subjects will have a frequency distribution that is then used to generate the deciles made up of 10 equal-sized quantiles.
In case any further guidance is needed, most suitably deciles are generated as described in the examples section below.

### SUBJECTS

The present invention may be applied to any subject from newborn onwards.

The present invention may be applied to adults or children.

Suitably the subject is 18 years or younger, suitably 16 years or younger, suitably 15 years or younger, suitably 7 years or younger, suitably 6 years or younger, suitably 5 years or younger, suitably 2 years or younger.

It should be noted that most immune systems function as "adult" from 2 to 5 years onwards. Thus, suitably the subject is at least 2 to 5 years old. Suitably the subject is at least 2 years old. Suitably the subject is at least 3 years, suitably at least 4 years, most suitably at least 5 years old.

Suitably the subject is a child.

Suitably the subject is 16 years or younger.

Suitably the subject is 15 years or younger.

Suitably the subject is 2 to 16 years old, suitably 3 to 16, suitably 4 to 16, suitably 5 to 16 years old.
Suitably the subject is 2 to 15 years old, suitably 3 to 15, suitably 4 to 15, suitably 5 to 15 years old.

Suitably the subject to betested has presented with at least one of the following symptoms: coughing, weight loss, sweating, swollen glands, and optionally sepsis.

The invention may be applied to intra-thoracic TB.
The invention may be applied to pulmonary T_{B}.
The invention may be applied to extra-pulmonary TB.

The invention may be applied to patients which are uninfected with HIV.
The invention may be applied to patients which are infected with HIV.

It should be noted that since the method is based on the immune response, that any subject with a CD4 count of 50 or fewer is unlikely to show a response. Thus, suitably the subject has a CD4 count of 51 or greater. For reference, a normal CD4 count in a healthy human is approximately 1000.

### DETECTION

Suitably the biomarkers described herein are detected by the suitable means in the art. For example, the biomarkers may be detected by one or more antibodies which specifically recognise said biomarkers.
For example, the biomarkers may be detected by an antibody or antigen binding fragment thereof as described above, wherein the antigen binding fragment thereof is selected from the group consisting of a Fab fragment, a Fab' fragment, a F(ab')2 fragment, a scFv, a Fv, a rIgG, and a diabody.

The mode of assessing binding of an antibody or antigen binding fragment thereof to detect the markers is a matter of operator choice. In case any guidance is needed, ELISA's could be used for each of the cytokines identified, for example one ELISA for each biomarker. Below are examples of suitable ELISA reagents.

ELISA's for thecytokines included in the signature of the invention may be obtained commercially from the following companies, with exemplary product names/details where appropriate:
Quantikine sandwich Elisa from R&D Systems UK, 19 Barton Lane, Abingdon Science Park, Abingdon, OX14 3NB, United Kingdom (Tel +44 (0)800 3734 15).

Human Platinum Elisa or high sensitivity Elisa from eBioscience, Ltd. (Ireland, United Kingdom), 2nd Floor, Titan Court, 3 Bishop Square, Hatfield, AL10 9NA, United Kingdom.

BD OptEIA kits from BD Biosciences, Edmund Halley Road, Oxford Science Park, Oxford, OX4 4DQ (Tel.: +44 1865 781 666; Fax: +44 1865 781 627).

As the skilled worker will be aware, individual ELISAs for each cytokine might be laborious, and/or require larger sample sizes. Therefore it is an advantage to carry out the detection in a multiplex or single sample where possible. This provides advantages such as low volume (particularly important for a paediatric test where lower volumes of blood/serum are desirable), and combination of the cytokines(less labour to complete the test).

There are numerous commercial suppliers of suitable multiplexing kit(s) useful to detect the biomarkers of the signature, for example:
MILLIPLEX MAP Human Cytokine/Chemokine Magnetic Bead Panel - Immunology Multiplex Assay, Catalogue number: HCYTOMAG-60K availablefrom Merck-Millipore, Suite 21, Building 6, Croxley Green Business Park, Watford, Hertfordshire WD18 8YH, United Kingdom.

Human Luminex Performance Assay Base Kit, Panel A [catalogue number LUH000] from R&D Systems UK, 19 Barton Lane, Abingdon Science Park, Abingdon, OX14 3NB, United Kingdom (Tel: +44 (0) 800 37 34 15).

Human Cytokine/ Chemokine/ Growth Factor Panel 1 (45 plex), (Catalogue number: EPX450-12171-901) from eBioscience, Ltd., (Ireland, United Kingdom), 2nd Floor, Titan Court, 3 Bishop Square, Hatfield, AL 10 9NA, United Kingdom.

Most suitably the antibodies used may be as in the commercially available Bio- Rad Human cytokine Th-1/Th-2 27-plex kit (catalogue number #M500KCAF0Y from Bio-Rad Laboratories Ltd., Bio-Rad House, Maxted Road, Hemel Hempstead, Hertfordshire, HP2 7DX, United Kingdom). Most suitably detection of thecytokines of the signature of the invention are detected/ assayed using this kit.

It is important to note that the prevailing view in the art is that stimulation of cells is required for use for analysis. The stimulation may be by presentation with bacteria, or may be by presentation with antigen or any other appropriate form of stimulation. However, it should be noted that these types of stimulations are all directed at analysing recall responses. It is an advantage of the invention that unstimulated samples are analysed. In particular, it is an advantage that the sample is from unstimulated blood.

In particular when studying the key panel of 8 biomarkers, suitably the invention omits astimulation step; suitably the invention does not comprise a stimulation step; suitably the invention excludes astimulation step.
In some embodiments a ninth or further marker may be employed - a stimulation step may be employed for such further marker(s) if appropriate.
Most suitably the invention omits a stimulation step. Most suitably the invention does not comprise a stimulation step. Most suitably the invention excludesa stimulation step.

### Nucleic acid detection

For example, the biomarkers may be detected in nucleic acid form, for example by detection of one or more mRNAs encoding the biomarkers.

If the skilled worker desires to read-out/ detect nucleic acids via a microarray approach, reference is made to Anderson et al 2014 (N Engl J Med. 2014 May 1;370(18):1712-23 'Diagnosis of childhood tuberculosis and host RNA expression in Africa.' ILULU Consortium; KIDS TB Study Group.)

mRNA technologies are suitably deployed in a laboratory setting.

In outline, mRNA detection may comprise the following steps:
- stabilisation of RNA- can be done with a specific reagent,
- extracted of RNA,
- transcription to cDNA,
- amplification,
- array,
- data read out.

Of course the person skilled in the art will realise that some steps are optional or may be combined, for example stabilisation/extraction may not be required if transcription can be performed directly on the sample. For example, array may not be required if the amplified material is assayed directly.

Thus in essence the required steps are:
- extraction of nucleic acid
- assay of nucleicacid todeterminemRNA expression level of markers of interest
- data read out.

For multiplex detection, suitably a fluorogenic oligonucleotide probe that is specific to the target gene/amplified target is used. Taqman probes are commonly used for multiplexing, but can also be used if multiplexing not required. Protocols are as stated by the manufacturer e.g. Applied Biosystems (5791 Van Allen Way, Carlsbad, California 92008, US).

Different dyes can be used for the fluorogenic probes; examples that may be useful depending on the buffer conditions and type of thermal cycler are shown in the table below (Table from Qiagen Inc):

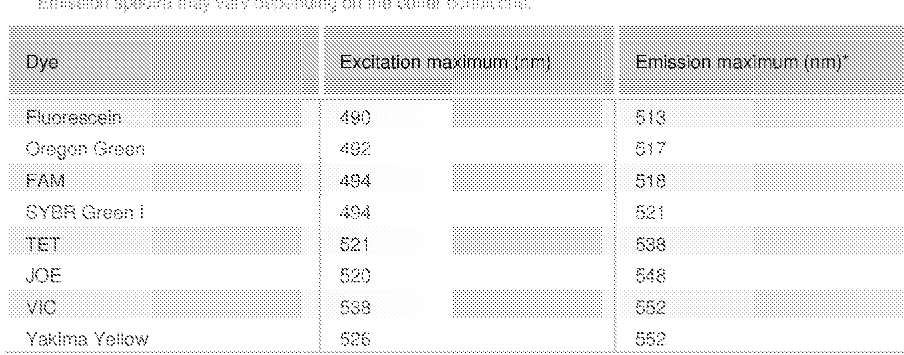

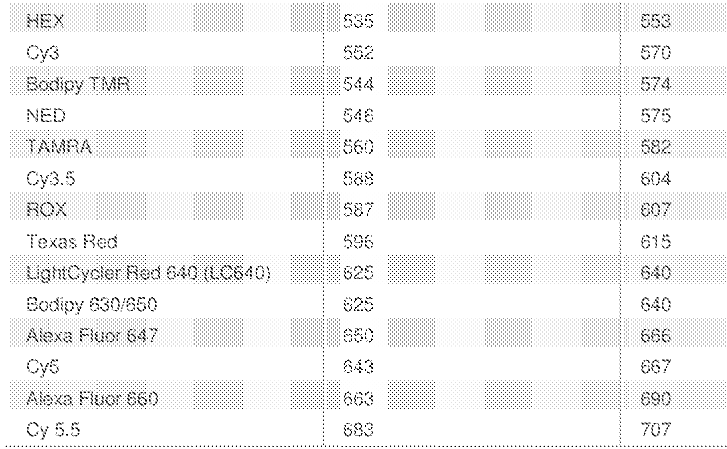

Protocols are well known in the art, for example using the StepOne and/or StepOne Plus Real Time PCR Systems according to manufacturer's instructions (Applied Biosystems (5791 Van Allen Way, Carlsbad, California 92008, US).

Other assays may be used if multiplexing was not desired, for example after reverse transcription, using dyes that bind double-stranded DNA and become florescent. For example SYBR green 1 (Qiagen Inc./Qiagen Ltd. Skelton House, Lloyd Street North, Manchester M15 6SH, UK) may be used.

Primer probe assays useful in the invention can be designed, or purchased predesigned, to the gene target of interest i.e. the biomarkers of the invention. For example, Applied Biosystems/ThermoFisher Scientific's (5791 Van Allen Way, Carlsbad, California 92008, US) protocol for SYBR green 1 custom design ("Design and optimization of SYBR Green assays") may be used, including the publicly available primer design tools discussed therein. This document is here by incorporated herein by reference specifically for the primer/probe design protocols and nucleic acid detection teachings.

I n case any further guidance is required, reference is made to the examples section below.

### DETERM INATION OF QUANTI LES/ DECI LES

Suitably converting each biomarker concentration determined into a decile value comprises the steps of:
(ci) comparing the concentration of each biomarker determined to a reference frequency distribution of concentrations of said biomarker; and
(cii) reading out the decile value from the frequency distribution for the concentration of said biomarker.

A "frequency distribution" shows asummarized grouping of data divided into mutually exclusive classes and the number of occurrences in a class. So it is possible to prepare a frequency distribution even with small numbers of datapoints such as 30 (e.g. exam scores of 30 children in aclass). The larger the number of subjects used (i.e. data points), the more representative the distribution will be of the true population i.e. the more normally distributed the frequency distribution will be. Biological variables e.g. weight, height, blood pressure, Haemoglobin concentration, electrolytes in blood, cytokine measurements etc. are usually skewed. Thus, to get a normally distributed curve for such biological variables using a normal histogram for example, very large numbers of data points are needed which may be problematic. Therefore, non-parametric methods such as Kernel, which is a data-smoothing density estimator, are used. This is especially helpful when we want to present the representation of distribution of such data with a reasonable good sample size e.g. of 100 or more.

Therefore, when 'frequency distribution' is mentioned herein, suitably this may comprise a non-parametric equivalent such as a non-parametric density estimator, such as a data-smoothing density estimator, most suitably a Kernel density estimator.

Considering Figures 2 to 9, the Y-axes are appropriately labelled 'Density' because they indicate the Kernel Density Estimate.

In more detail, the Kernel frequency distribution isadata-smoothing statistical approach to displaying frequency distribution. Unlike the ordinary 'histogram', it is a non-parametric method which makes it very appropriate for our type of data, which like most biological data, does not follow the normal Gaussian distribution. The basic histogram displays frequency in number or proportion and the problems witll histograms include the fact that it is not smooth and depends on the width of the bins (i.e. the bars) and end point of the bins which can be arbitrarily chosen. However, Kernel density estimate removes the dependence on end point of the bins by assuming no gap in the interval of cytokine measurements within a specified range, giving a smooth density estimate. The Y-axis of Figures 2 to 9 can simply be summarised as: "the probability density function of each respective marker."

Suitably the reference frequency distribution (or Kernel Density Estimate) is generated by measuring the concentration of the biomarker in a number of subjects, for example a minimum of 100 subjects, and compiling those measurements into a frequency distribution (or Kernel Density Estimate).

Alternatively the frequency distributions (Kernel Density Estimates) presented in Figures 2 to 9 herein may be used.

Suitably step (c) converting each concentration determined in (b) into a decile value comprises the steps of:
(ci) comparing the concentration of each biomarker determined in (b) to the corresponding reference frequency distribution or Kernel Density Estimate of concentrations of said biomarker selected from Figures 2 to 9; and
(cii) reading out the decile value from the frequency distribution or Kernel Density Estimate for the concentration of said biomarker.

In one embodiment, decile/quantile cutoffs may be augmented or replaced by absolute cut-offs expressed as absolute concentrations of the biomarker(s) in the sample. Exemplary absolute cut-off values are provided in the table below:

| **Biomarker** | **Specific quantile cut-off value** | **Concentration range for specific quantile (pg/ml)** | **Absolute cut-off concentrations i.e. ≥ XX** |
|---|---|---|---|
| IL-1ra | 3 | 76.0 - 99.5 | 76.0 |
| IL6 | 6 | 759.0 - 1203.0 | 759.0 |
| IL-7 | 8 | 167.0 - 269.0 | 167.0 |
| IL-8 | 9 | 20196.0 - 25900.0 | 20196.0 |
| IL-12p70 | 9 | 596.0 - 776.0 | 596.0 |
| FGF-basic | 3 | 125.5 - 145.0 | 125.5 |
| IP-10 | 4 | 3096.5 - 4537.0 | 3096.5 |
| VEGF | 9 | 3378.0 - 5381.0 | 3378.0 |

In one embodiment, decile/quantile cutoffs may be augmented or replaced by the concentration range for the specific quantile of interest expressed as the range of absolute concentrations of the biomarker(s) in the sample. Exemplary concentration ranges are provided in the table above.

### POINT OF CARE TEST

In many embodiments, the skilled operator may choose to analyse the concentrations of the markers in a laboratory or test facility.

The invention may also be applied as a point of care test. Suitably the invention may also be applied as a bedside test.

When the invention is a point of care/bedside test, suitably the sample is blood or plasma. When the invention isapoint of care/bedside test, suitably the markers are analysed in protein form.

When the invention is a point of care/bedside test, suitably the detection is immunological detection.

When the invention is a point of care/bedside test, suitably the test is the a format of a lateral flow assay.

### PREDICTIVE VALUED/APPLICATIONS OF THE METHOD

The table below provides additional results of predictive values of the biosignature. We show and compare the PPV and NPV of the biosignature to prior art methods. We further show it has demonstrable comparable performance to the gold standard of culture. The high specificity and the positive predictive value of the invention lends itself to change treatment decisions. This enables accurate prescription for TB detected according to the invention. This also avoids waste of resources in prescription of unnecessary drugs. This further demonstrates the utility of the invention.

| **Diagnostic accuracy of tests relative to a 'composite reference standard'** | | | | |
|---|---|---|---|---|
| | 'All TB diagnosis' as a Composite reference standard | | | |
| | Sensitivity (%) (95% CI) | Specificity (%) (95% CI) | PPV (%) (95% CI) | NPV (%) (95% CI) |
| **Chest X-ray (prior art)** | 76 (62 - 86) | 34 (26 - 43) | 34 (25 - 43) | 76 (62 - 87) |
| **Clinical algorithm (prior art)** | 15 (7 - 28) | 99 (95 - 100) | 89 (52 - 100) | 73 (65 - 79) |
| **Culture (prior art)** | 36 (23 - 50) | 100 (97 - 100) | 100 (82 - 100) | 78 (71 - 84) |
| **Biosignature (invention)** | 23 | 99 | 92 | 75 |
| | (12 - 36) | (95 - 100) | (64 - 100) | (67 - 81) |

### FURTHER APPLICATIONS

The key set of 8 biomarkers are advantageously assessed from unstimulated samples. However, in some embodiments it may be helpful to further assess a ninth or further marker; suitably such a ninth or further marker comprises a stimulated marker such as EC-stimulated VEGF. Suitably the cutoff for EC-stimulated VEGF is decile 2. For the avoidance of doubt, details of the 'VEGF' biomarker of EC-stimulated VEGF areas above in the key panel of 8 biomarkers of the invention ('VEGF').

A method for aiding the diagnosis of TB in a subject, the method comprising;
(a) providing a sample from said subject, said sample being selected from the group consisting of: blood, serum and plasma;
(b) determining the concentration in said sample of the following biomarkers: IL-1ra, IL6, IL-7, IL-8, IL-12p70, FGF-basic, IP-10, and VEGF;
(c) converting each biomarker concentration determined in (b) into adecilevalue; and
(d) converting each decile value into a binary presenceor absence by comparing the decile values of (c) to the following specific quantile cut-off values:

| **Biomarker** | **Specific quantile cut-off value** |
|---|---|
| IL-1ra | 3 |
| IL6 | 6 |
| IL-7 | 8 |
| IL-8 | 9 |
| IL-12p70 | 9 |
| FGF-basic | 3 |
| IP-10 | 4 |
| VEGF | 9 |

wherein a decile value matching or exceeding the specific quantile cut-off value is converted into the binary presence of the biomarker, and a decile valuelower than the specific quantile cut-off valueis converted into the binary absence of the biomarker;
wherein detecting the presence of each of said biomarkers indicates an increased likelihood that the subject has TB.

A method for differentiating TB from OD in a subject, the method comprising; carrying out steps (a) to (d) above
wherein detecting the presence of each of said biomarkers indicates that the subject has TB.

A method of collecting information useful in diagnosis of TB in a subject, the method comprising:
carrying out steps (a) to (d) above
wherein detecting the presence of each of said biomarkers identifies the subject as having TB.

A method for the diagnosis of TB in a subject, the method comprising;
carrying out steps (a) to (d) above
wherein detecting the presence of each of said biomarkers provides the diagnosis that the subject has TB.

A method for selecting a subject to receive treatment for TB, the method comprising; carrying out steps (a) to (d) above
wherein detecting the presence of each of said biomarkers selects said subject to receive said treatment.

A method comprising the steps of selecting a subject to receive treatment for TB by; carrying out steps (a) to (d) above
wherein detecting the presence of each of said biomarkers selects said subject to receive said treatment; and administering said treatment to said subject.

A method of treating TB in a subject comprising administering a regimen of 2HRZE/4HR (2 months HRZE followed by 4 months HR wherein H = isoniazid, R = rifampicin, Z = pyrazinamide, E = ethambutol) to a subject determined to have each of the following biomarkers: IL-1ra, IL6, IL-7, IL-8, IL-12p70, FGF-basic, IP-10, and VEGF. The invention also relates to said method further comprising testing the subject prior to the administering step to determine that the subject has the following biomarkers: IL-1ra, IL6, IL-7, IL-8, IL-12p70, FGF-basic, IP-10, and VEGF. Suitably testing is carried out by carrying out steps(a) to (d) above.

In so far as the embodiments of the invention described above are implemented, at least in part, using software-controlled data processing apparatus, it will be appreciated that a computer program providing such software control, and a storage medium by which such a computer program is stored, are envisaged as aspects of the present invention. Clearly in several of the methods or processes of the invention, one step (typically step (a)) comprises providing a sample from the subject - clearly that step would not typically be performed using software-controlled data processing apparatus; suitably that step is manually executed, or omitted, in embodiments implemented using software-controlled data processing apparatus.

Thus the invention relates to an apparatus such as a computer comprising logic, circuitry or code configured to carry out the method as described above.

Thus the invention relates to a computer program product operable, when executed on a computer, to perform the method as described above.

Further particular and preferred aspects are set out in the accompanying independent and dependent claims. Features of the dependent claims may be combined with features of the independent claims as appropriate, and in combinations other than those explicitly set out in the claims.

Where an apparatus feature is described as being operable to provide a function, it will be appreciated that this includes an apparatus feature which provides that function or which is adapted or configured to provide that function.

### BRIEF DESCRIPTION OF THE DRAWINGS

Embodiments of the present invention will now be described by way of example, with reference to the accompanying drawings, in which:
Figure 1 shows optimal LASSO models with cytokine covariates, adjusted for age and origin. Panels a, b, c: Optimal LASSO model, adjusted for age and origin, determined by 5-fold cross validation in training set. Panels d, e, f: box-and-whisker plot showing probability of TB disease in the bacteriologically-confirmed TB, clinically diagnosed TB and OD subjects in the training set as predicted by the identified biosignature. Panels g, h, i: AUC showing discriminating ability of identified biosignature to classify confirmed TB from OD in the independent test set
Figure 2 shows a frequency distribution for IL-1ra
Figure 3 shows a frequency distribution for IL-6
Figure 4 shows a frequency distribution for IL-7
Figure 5 shows a frequency distribution for IL-8
Figure 6 shows a frequency distribution for IL-12p70
Figure 7 shows a frequency distribution for FGF-basic
Figure 8 shows afrequency distribution for IP-10
Figure 9 shows a frequency distribution for VEGF
Figure 10 shows a lateral flow device.
Figure 11 shows the multiplex detection format of a lateral flow device.

### EXAMPLES

### Methods

### Brief cohort description

Children aged less than 15 years who were exposed to an adult infectious TB case in the household setting were actively traced and screened for symptoms suggestive of TB disease in the respective households. Those with suspected intrathoracic TB disease thereafter had further detailed clinical evaluation and investigations to ascertain their TB disease status. A total of 173 child TB contacts with suspected intrathoracic TB disease, prospectively recruited both in The Gambia (n=150) and United Kingdom (n=23), were included in the biosignature discovery experiments using an immuno-epidemiological approach.

### Whole blood stimulation assay (WBA)

For the Gambia cohort, a WBA was set up at the recruitment within four hours of venepuncture. 100µl of undiluted heparinised whole blood was incubated in duplicates with *M.tb* antigens ESAT-6/CFP-10 fusion protein (EC; 10µg/ml final concentration; kindly provided by Professor Tom Ottenhoff, Leiden University Medical Center, The Netherlands) and positive (PHA-L, Sigma Chemicals, UK; 10µ g/ml final concentration) and negative (RPMI 1640 medium; BioWittaker, Verviers, Belgium) controls. After overnight incubation at 37°C with 5% C0₂, supernatants were harvested, duplicates pooled and stored at -20°C prior to analysis. Samples were added to this cohort from an equivalent set up of a household contact study in the UK, conducted by BK. For the children from the UK cohort, we had also obtained the relevant demographic and clinical data and derived supernatants from IGRA (Quantiferon-TB Gold In-Tube (QFT-G) test (Cellestis, Australia). Similar to our in-house assay, this commercially available in-vitro IFN-γ release assay uses stimulation of fresh whole blood in three separate tubes containing M.tb antigens (ESAT-6, CFP10 and TB 7.7), positive (Phytoheamaglutinin-L) and negative (Nil) controls respectively. These samples were shipped frozen to The Gambia for joint analysis.

The WBA supernatants from the Gambian children and the QFT supernatants from the children from the UK cohort were used for a multiplex cytokine detection assay (MCA). The MCA was carried out on site in the MRC TB Immunology laboratory in The Gambia, with the Gambian and UK samples randomly distributed over the multiplex plates.

### Multiplex cytokine detection assay (MCA)

We carried out a comprehensive MCA by Luminex using the unstimulated and EC-stimulated WBA supernatants of the Gambian children and QFT supernatants (from antigen and nil QFT tubes) of the children from the UK cohort. Culture supernatants were analysed using the Bio- Rad Human cytokine Th-1/Th-2 27-plex kit according to the manufacturer's instruction and as described previously [1]. Cytokines assessed were: IL-1b, IL-1ra, IL-2, IL-4, IL-5, IL-6, IL-7, IL-8, IL-9, IL-10, IL-12p70, IL-13, IL-15, IL-17, Eotaxin, FGF basic, G-CSF, GM- CSF, IFNγ, IL-10, MCP-1(MCAF), MIP-1a, MIP-1b, PDGF-bb, RANTES, TNFα and VEGF. Following pre-wetting of the filter plate, 50µl of bead suspension was added to each well and washed twice. 50µl of samples and standards, tested singly and in duplicates respectively, were then added and the plate sealed and shaken for 30 seconds at 1100rpm and then incubated for one hour at 300rpm. The plate was washed three times, 25µl of pre-diluted detection antibody was added and the plate shaken and incubated for 30 minutes at 300rpm in the dark. After washing, 50µl of 1 x streptavidin-PE was added to each well and incubated for 10 minutes with shaking at 300rpm. The plate was again washed and resuspended in 125µl of the assay buffer, sealed, mixed and immediately read on the Bio-plex analyser using Bioplex manager software (version 4.0; Bio-Rad, USA) and a low photomultiplier tube (PMT) setting. Cytokine concentrations below the level of detection - reported as 'OOR' in the Bioplex software - were calculated as zero in the analysis.

### Statistical analysis

We analysed the data obtained from the multiplex cytokine assay (MCA) of unstimulated and EC-stimulated whole blood culture supernatants for the identification of the host-specific multicytokine biosignature associated with TB in children. For this analyses, the unstimulated and antigen-specific cytokine responses from the 27-plex MCA were analysed as separate variables. We randomly assigned the study subjects into a training set (80% of subjects) and an independent test set (20%). We then used Generalized Linear Model (GLM) applying Least Absolute Shrinkage and Selection Operator (LASSO) penalty to fit logistic regression models in the training set adjusting for age in years and origin of sample, initially with bacteriologically confirmed TB (gold standard) compared to other respiratory diseases mimicking TB but not TB (OD group) as the binary outcome variable. The LASSO model applies a maximum penalised likelihood to the absolute size of the regression coefficients, shrinking them towards zero i.e. an L1 norm penalty is applied to the regression coefficients. This procedure results in both variable selection (some regression coefficients equal zero) and estimates of non-zero regression coefficients shrunk towards zero. This methodology is suitable to our cytokine data in which there were very many measures, many of which could potentially be highly correlated.

We fitted the cytokine covariates as categorical and continuous variables and as a combination of both. Categorical cytokine covariates were constructed by a split of the cytokine values into deciles by dividing the frequency distribution of each cytokine value into 10 equal-sized quantiles, which were then fitted into the model as 10 binary variables for each cytokine using each of 10 quantiles as a cut off. The optimal LASSO model was determined using a 5-fold cross-validation in the training set, which was subsequently applied to classify bacteriologically confirmed TB from OD in the independent test set naive of origin. The optimal model was defined as the model with the highest penalty parameter ('lambda) resulting in the smallest prediction error and the best mean cross-validation AUC. The cross-validation process accounts for, and replaces the classical method of adjusting for multiple testing. In addition, it naturally protects against over-fitting and it is a way of assessing how a model will generalise to an independent dataset. The prediction performance of the optimal LASSO model was evaluated by estimating prediction probabilities for TB and area under the receiver operating characteristics curves (AUC).

### Results

Overall, 53 children from the combined cohorts were diagnosed with TB and started on standard TB treatment; 24 had bacteriologically-confirmed TB and 29 had TB diagnosed based on clinical and radiological features with no positive microbiological tests. One hundred and twenty were diagnosed and treated for OD. In detail, thirty-five of the 150 Gambian children had TB, comprising 16 bacteriologically-confirmed and 19 clinical diagnosed TB cases, while 115 had OD. Of the 23 children from the UK, 18 were diagnosed with TB (8 confirmed and 10 clinically diagnosed TB) while 5 had OD. None of the children recruited from The Gambia or UK was HIV infected. Table 1 shows that the distribution of the baseline profiles of the children from Gambia and UK were comparable.

**Table 1: Demographic profile of children by origin**

| Characteristics | **Total** | **The Gambia** | **United Kingdom** |
|---|---|---|---|
| | N = 173 | N= 150 | N=23 |

| **Age**: | | | |
|---|---|---|---|
| Years, Median (IQR) | 6 (3 - 9) | 6 (3 - 9) | 7 (3 - 12) |
| < 5 years, n/N (%) | 65/173 (38) | 55/150 (37) | 10/23 (43) |
| | | | |

| **Gender**: | | | |
|---|---|---|---|
| Male, n/N (%) | 88 (51) | 76 (51) | 12 (52) |
| | | | |

| **HIV test:** | | | |
|---|---|---|---|
| Positive, n/N (%) | 0 | 0 | 0 |
| | | | |

| **BCG Scar:** | | | |
|---|---|---|---|
| Present, n/N (%) | 117/164 (71) | 99/141 (70) | 18/23 (78) |
| | | | |

| **TST**: | | | |
|---|---|---|---|
| ≥ 10mm, n/N (%) | 98/171 (57) | 82/149 (55) | 16/22 (72) |
| | | | |

| **IGRA:** | | | |
|---|---|---|---|
| Positive, n/N (%) | 120/173 (69) | 103/150 (69) | 17/23 (74) |

### Pattern of cytokine and chemokine production in confirmed TB vs OD

The concentrations of cytokines and chemokines obtained by a 27-plex multiplex cytokine analysis of unstimulated supernatants and EC-stimulated supernatants from children with bacteriologically confirmed TB were compared with the levels in children with OD by multivariable linear regression analyses, adjusting for age in years and origin. The unstimulated and EC-specific values (i.e. EC-stimulated *minus* unstimulated negative control values) for each cytokine or chemokine were analysed as separate variables.

**Table 2. Mean difference in concentration (pg/ml) of the 27-markers: Confirmed TB vs OD**

| **Analytes** | **Unstimulated values (pg/ml)** | | | **ESAT-6/CFP-10-speciric values (pg/ml)** | | |
|---|---|---|---|---|---|---|
| | Confirmed TB vs OD | | | Confirmed TB vs OD | | |
| | Mean difference | 95% CI | P-value^{Ω} | Mean difference | 95% CI | P-value^{Ω} |
| IL-1b | 307.7 | -961.8 - 1577.1 | 0.633 | 41.3 | -4025.6 - 4108.3 | 0.984 |
| **IL-1ra** | **118.2** | **13.4 - 222.9** | **0.027** | 336.9 | -359.5 - 1033.4 | 0.340 |
| IL-2 | 43.9 | -71.7 - 159.6 | 0.454 | **4995.1** | **1981.2 - 8009.0** | **0.001** |
| IL-4 | 58.8 | -2.3 - 119.9 | 0.059 | 76.4 | -52.4 - 205.2 | 0.243 |
| IL-5 | 36.8 | -17.8 - 91.4 | 0.185 | -39.9 | -670.3 - 590.3 | 0.900 |
| IL-6 | 1289.8 | -1193.7 - 3773.3 | 0.306 | -555.1 | -4207.1 - 3097.0 | 0.764 |
| **IL-7** | **181.8** | **90.0 - 273.6** | **0.000** | **-82.1** | **-140.9 to -23.3** | **0.007** |
| IL-8 | 2555.1 | -388.3 - 5498.6 | 0.088 | -1800.4 | -5554.9 - 1954.1 | 0.345 |
| IL-9 | 81.6 | -28.1 - 191.1 | 0.144 | -61.9 | -281.8 - 157.9 | 0.578 |
| IL-10 | 5 1.2 | -140.7 - 243.2 | 0.598 | 28.1 | -3357.7 - 3413.9 | 0.987 |
| **IL-12p70** | **153.5** | **43.0 - 263.9** | **0.007** | 7.1 | -158.9 - 173.0 | 0.933 |
| **IL-13** | **129.1** | **26.7 - 231.6** | **0.014** | 728.2 | -331.4 - 1787.8 | 0.176 |
| **IL-15** | **1258.5** | **468.4 - 2048.6** | **0.002** | -237.7 | -928.7 - 453.3 | 0.498 |
| **IL-17** | **116.3** | **11.9 - 220.7** | **0.029** | 122.6 | -20.3 - 265.5 | 0.092 |
| **Eotaxin** | **214.2** | **75.5 - 352.9** | **0.003** | 13.4 | -138.5 - 165.2 | 0.862 |
| **FGF-** | **114.9** | **13.6 - 216.1** | **0.026** | 65.5 | -59.7 - 190.8 | 0.303 |
| **GCSF** | **222.9** | **51.3 - 394.6** | **0.011** | 1009.0 | -793.6 - 2811.6 | 0.270 |
| GMCSF | 41.4 | -185.6 - 268.4 | 0.719 | 236.1 | -81.0 - 553.2 | 0.143 |
| **IFN-γ** | **81.4** | **4.8 - 158.0** | **0.038** | 2148.9 | -1144.4 - 5442.4 | 0.199 |
| **IP-10** | **4631.9** | **877.2 - 8386.7** | **0.016** | -434.3 | -4602.8 - 3734.2 | 0.837 |
| MCP1- | 4739.8 | -314.5 - 9794.1 | 0.066 | -3830.4 | -9061.8 - 1400.9 | 0.150 |
| MIP1a | 2721.4 | -337.5 - 5780.3 | 0.081 | -939.9 | -4887.5 - 3007.8 | 0.639 |
| **MIP1b** | **4400.8** | **364.0 - 8437.5** | **0.033** | -3997.5 | -8244.7 - 249.8 | 0.065 |
| PDGF | 326.1 | -1053 - 1705.3 | 0.641 | 437.8 | -575.8 - 1451.2 | 0.395 |
| RANTES | -106.3 | -1446.0 - 1233.4 | 0.876 | 273.5 | -943.5 - 1490.6 | 0.657 |
| TNF-α | 273.8 | -1.7 - 549.3 | 0.051 | -399.0 | -4033.0 - 3234.9 | 0.828 |
| VEGF | **1977.7** | **1116.1 - 2839.2** | **0.000** | -491.6 | -1224.0 - 240.8 | 0.187 |

| | | | | | | |
|---|---|---|---|---|---|---|
| ^{Ω}*Adjusted for age in years and origin* *95% CI = 95% confidence interval of mean difference* | | | | | | |

As shown in Table 2, of the 27 cytokines and chemokines analysed, the unstimulated concentrations of IL1ra, IL7, IL12p70, IL13, IL15, IL17, Eotaxin, basic FGF, GCSF, IFN-γ, IP10, MIP1b and VEGF were significantly higher in children with confirmed TB compared to children with OD. Of the EC-specific concentrations of all the analytes, only IL2 and IL7 were significantly different between the two groups. Furthermore, we found that the concentrations in unstimulated samples were significantly higher for all the analytes in children from the UK compared to Gambian children regardless of age or diagnosis (p-value <0.001 for all), with the exception of unstimulated-IL7 value in which there was no significant difference (data not shown). On the contrary, the EC-specific concentrations of the analytes were significantly lower in children from the UK compared to Gambian children regardless of age and diagnosis (p-value <0.001 in all), with the exception of EC-specific concentrations of IL2, IL5, IL7, IL13, IFN-γ and RANTES in which there were no significant differences.

### Identification of a host specific biosignature for the diagnosis of childhood TB

Using GLM with LASSO penalty to fit a binary logistic regression model, adjusting for age in years and origin and with 5-fold cross-validation in the training set, a combination of nine categorical cytokines optimally predicted TB or OD with a mean cross-validation AUC of 0.82. Each of the nine cytokines is a binary variable with a cut-off value.

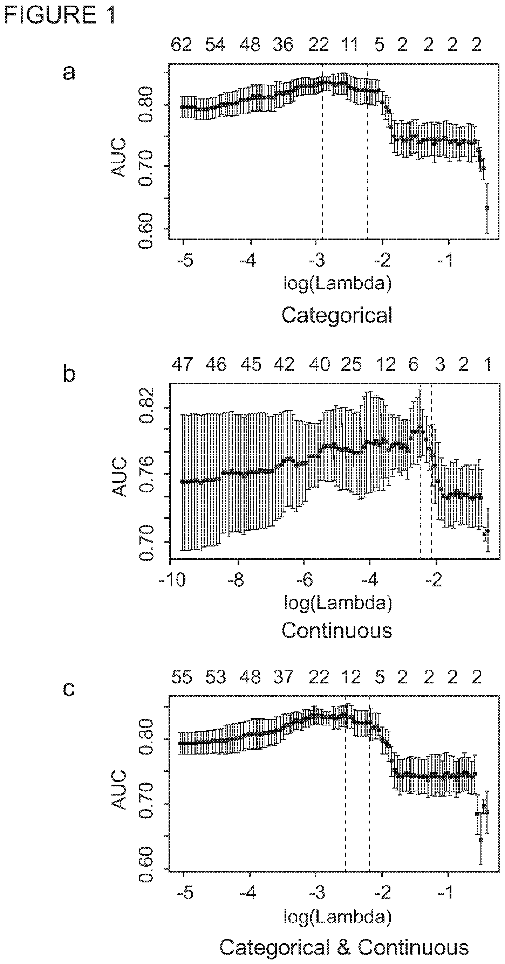

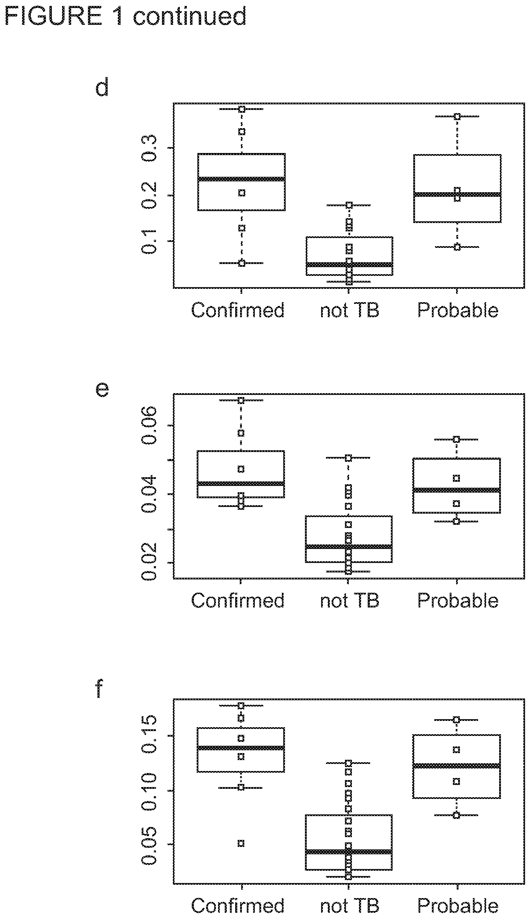

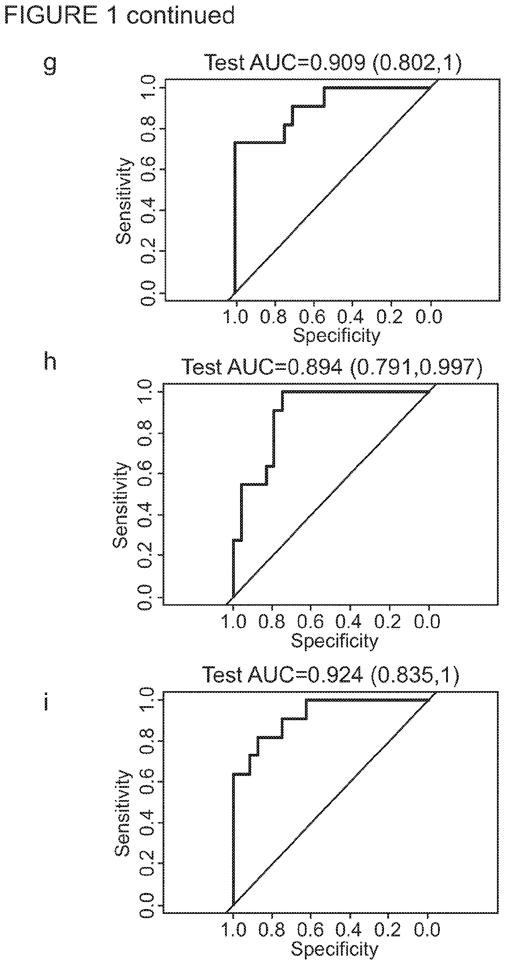

The cytokines - with the specific quantile cut-off value for each in bracket - were: unstimulated IL-1ra (3), IL6 (6), IL-7 (8), IL-8 (9), IL-12p70 (9), FGF-basic (3), IP-10 (4), VEGF (9) and EC-stimulated VEGF (2). When applied to the independent test set, this model reliably classified confirmed TB from OD with an AUC of 0.91(95% CI 0.80 -1.0) as depicted graphically in Figure 1.

### Diagnostic accuracy and added value of the multicytokine biosignature

The quantile specific cut-off values for each cytokine in the biosignature enabled us to convert the biosignature into a binary - positive or negative - test. We investigated the diagnostic accuracy of the biosignature as two separate variables i.e. *"biosignature 1*" (combination of all the 9 identified cytokines) and *"biosignature 2"* (combination of only the 8 cytokines from unstimulated supernatants). When we compared the results of the novel multicytokine biosignatures to the *disease certainty classification* (i.e. bacteriologically-confirmed TB (= confirmed TB), clinically diagnosed TB (= probable TB) and other respiratory diseases but not-TB (OD) as defined by the WHO [2], *'biosignature1'* was positive in 8% of confirmed TB and 7% of clinically diagnosed TB cases. However *'biosignature2'* had a relatively higher sensitivity with positive results in 21% and 24% of confirmed and clinically diagnosed TB cases respectively. Both biosignature versions were negative in 119 of 120 OD cases giving a very high specificity of 99.2% (Table 3).

**Table 3: Performances of biosignatures relative to TB disease certainty classification**

| **WHO Revised TB Case Definition*** | | | |
|---|---|---|---|
| | Confirmed TB (N = 24), n (%) | Clinically diagnosed TB | OD (not-TB) (N = 120), n (%) |
| *'Biosignature1*' + | 2 | 2 | 1 (0.8) |
| *'Biosignature1' -* | 22 | 27 | 119 (99.2) |
| | | | |

| | Confirmed TB (N = 24), n (%) | Clinically diagnosed TB | OD (not-TB) (N = 120), n (%) |
|---|---|---|---|
| *'Biosignature2'* + | 5 | 7 | 1 (0.8) |
| *'Biosignaiure2*' - | 19 | 22 | **119 (99.2)** |

| | | | |
|---|---|---|---|
| + = *test positive;* - = *test negative;* % = *column percentage i.e. n*/*N* (* W.H.O. Definitions and reporting framework for Tuberculosis - 2013 revision, Geneva, Switzerland. www.who.int/iris/bitstream/10665/79199/1/9789241505345_eng.pdf [Accessed 03 December 2014]) | | | |

Using a composite reference standard of all children diagnosed with active TB disease, *'hiosignature2'* derived from only markers in unstimulated supernatants was positive in 12 of all the 53 children diagnosed with active TB disease giving a sensitivity of 23% (95% CI 12 -36). As individual tests, the sensitivity of *'biosignature2'* was significantly higher than that of smear microscopy (5.7%; p-value = 0.020) but comparable to that of *M.tb* culture (35.9%; p-value = 0.127). The combination of *'biosignature2'* and smear microscopy were positive in 15 of 53 children with active TB disease giving a sensitivity of 28.3%, which was significantly higher than the sensitivity of smear microscopy alone (5.7%; p <0.001). Similarly, *'biosignature2'* combined with *M.tb* culture had a sensitivity of 49.1%, which was significantly higher than the sensitivity of *M.tb* culture used alone (35.9%; p<0.001). The sensitivity of *'biosignature2'* combined with *M.tb* culture was significantly higher than that of *'biosignature2'* combined with microscopy (p < 0.001), but comparable to the sensitivity of the combination of *'biosignature2',* microscopy and *M.tb* culture (p =0.320). The use of *'biosignature2'* in combination with these diagnostic tests did not result in any change in the specificity of the tests.

### Summary

Since host immune factors such as IFN-γ have been shown to be important, but insufficient to confirm or exclude TB [3, 4], the aim of this study was to investigate cytokines other than IFN-γ that may help to differentiate TB from OD in Gambian and UK children, since the distinction of these two clinical presentations is important to initiate the right therapy. Previous studies have reported other cytokines such as TNF-α, IL-12(p40), IL-6, IL-10, IL-18 and IL-17, FGF and VEGF that have been found to be important in the immune response against *M.tb* and/or in distinguishing TB from OD [1, 5, 6].

We identified a unique, quantile-specific, 9-cytokine biosignature that optimally distinguished bacteriologically-confirmed TB from OD irrespective of age and origin of the children. The biosignature also predicted the probability of active TB disease in children with clinically-diagnosed TB that was comparable to that of bacteriologically-confirmed TB cases. Specifically, we used an age and origin adjusted LASSO regression model to identify a quantile-specific combination of unstimulated IL-1ra, IL-6, IL-7, IL-8, IL-12p70, basic FGF, IP-10, VEGF and EC-stimulated VEGF that optimally distinguished between bacteriologically-confirmed TB and OD with an AUC of 0.91 in an independent test set. The performance of this biosignature was regardless of sensitization to *M.tb* in the clinical outcome groups, while eight of the 9-cytokines in the biosignature were from unstimulated supernatants. A major strength of our study is the prospective approach used in an exclusively paediatric active case finding study setting while the identified biosignature in our cohort contains cytokines known to be closely associated with TB immunity.

We investigated separately the diagnostic accuracy of the 8-cytokines biosignature comprising only the markers from unstimulated supernatants, and our full 9-cytokine biosignature by comparing their results to *disease certainty classifications* according to WHO case definitions and a composite reference standard of all children diagnosed with active TB disease. We found that while the unstimulated 8-cytokine biosignature had a relatively higher sensitivity than the full 9-cytokine biosignature, both biosignature versions distinguished active TB disease from OD with a very high specificity of 99.2%. The unstimulated 8-cytokine biosignature detected a comparable number of TB cases among all children diagnosed with active TB disease in our study relative to *M.tb* culture, and demonstrated substantial added value when combined with routine TB diagnostic tests. It showed a comparably higher specificity but a lower sensitivity relative to a risk score based on a 51-whole blood gene transcript that was identified in a multi-country childhood TB biomarker study in south and eastern Africa, as well as a three marker combination of TNF-α, IL-12(p40) and IL-17 in antigen stimulated whole blood supernatants of adult Gambians [1, 7]. However, the specificity of this paediatric biosignature is comparable to that of the combination of IL-13, FCF and IFN-γ in *ex vivo* sputum samples in another study in adults in The Gambia, which resulted in 96% correct classification of consecutively recruited culture-confirmed TB cases from OD with a sensitivity of 85% and specificity of 96% [6].

A number of factors makes this unstimulated multicytokine biosignature a particularly promising approach for use in high TB burden countries. First, the quantile-specific cut-off values for each of the component cytokines mean the readouts can be easily converted into a binary test with either a positive or negative result, which makes it more easily interpretable. Secondly, this multicytokine biosignature, derived from only markers in unstimulated supernatants, had similar epidemiological properties to *M.tb* culture but is potentially not subject to the same time delay or risk of contamination as culture. Thirdly, it has a demonstrable potential to reduce presumptive treatment of TB disease in children in primary care settings in developing countries where TB diagnosis is mostly based on the use of smear microscopy, as well as at referral centres with X-ray, Xpert and/or culture facilities. This is because of the substantial increases in the number of children who would be deemed to have active TB disease when used in combination with the routine diagnostic tests. Fourthly, this unstimulated multicytokine biosignature could potentially be measured directly in serum or plasma samples without the additional cost, training or infrastructure needed for antigen stimulation and incubation in the laboratory. Thus the 8-cytokine biosignature is the most preferred embodiment of the invention.

### Example 2: Nucleic acid based detection

In this example we describe processing of samples to illustrate the application of the invention/gene signature in aiding diagnosis of TB (such as childhood TB) via nucleic acid detection, such as RNA (mRNA) detection.

### 1. Sample collection:

The blood sample is collected into a tube containing a stabilising agent for RNA, such as a PaxGene tube or tempus tube.

Alternatively trizol is added to sample.

Such sample may be stored in fridge or freezer until processing.

If stored in the fridge, the storage time is days, if stored in the freezer the storage time can be months.

### 2. Sample processing

Depending on the collection tube and stabilising agent, suitable commercially available RNA extraction kit(s) are selected and used according to the manufacturer's instructions.

In this example, Qiagen kits containing spin columns and wash buffers for RNA extraction are used.

Total RNA including micro RNA is extracted using these established methods.

### 3. Reverse transcription

In order to obtain DNA which can be amplified, a transcription reaction needs to first convert mRNA to cDNA. This is done by addition of RT random primers, dNTPs, Reverse Transcriptase and RT buffers in the appropriate amounts, followed by thermal cycling incubation as is known in the art.

In this way, the RNA is reverse transcribed into DNA.

### 4. Amplification

cDNA can then be amplified using primers and/or probes specific for the transcripts of interest of the biomarkers as described above, suitably together with primers and/or probes specific for reference genes for normalisation.

For convenience, in this example primers and/or probes for each cytokine in question, internal controls and endogenous control genes are added to a mastermix along with the cDNA template, and triplicate PCR reactions in either single-plex or multi-plex are carried out using a real-time PCR instrument.

### References:

1. Sutherland JS, de Jong BC, Jeffries DJ, Adetifa IM, Ota MO. Production of TNF- alpha, IL-12(p40) and IL-17 can discriminate between active TB disease and latent infection in a West African cohort. PloS one 2010: 5(8): e12365.
2. W.H.O. Definitions and reporting framework for Tuberculosis - 2013 revision., Geneva, Switzerland. www.who.int/iris/bitstream/10665/79199/1/9789241505345_eng.pdf[Accessed 03 December 2014], 2013.
3. Kaufmann SH. Fact and fiction in tuberculosis vaccine research: 10 years later. The Lancet infectious diseases 2011: 11(8): 633-640.
4. Flynn JL, Chan J, Triebold KJ, Dalton DK, Stewart TA, Bloom BR. An essential role for interferon gamma in resistance to Mycobacterium tuberculosis infection. The Journal of experimental medicine 1993: 178(6): 2249-2254.
5. Algood HM, Chan J, Flynn JL. Chemokines and tuberculosis. Cytokine Growth Factor Rev 2003: 14(6): 467-477.
6. Ota MO, Mendy JF, Donkor S, Togun T, Daramy M, Gomez MP, Chegou NN, Sillah AK, Owolabi O, Kampmann B, Walzl G, Sutherland JS. Rapid diagnosis of tuberculosis using ex vivo host biomarkers in sputum. The European respiratory journal : official journal of the European Society for Clinical Respiratory Physiology 2014: 44(1): 254-257.
7. Anderson ST, Kaforou M, Brent AJ, Wright VJ, Banwell CM, Chagaluka G, Crampin AC, Dockrell HM, French N, Hamilton MS, Hibberd ML, Kern F, Langford PR, Ling L, Mlotha R, Ottenhoff TH, Pienaar S, Pillay V, Scott JA, Twahir H, Wilkinson RJ, Coin LJ, Heyderman RS, Levin M, Eley B. Diagnosis of childhood tuberculosis and host RNA expression in Africa. The New England journal of medicine 2014: 370(18): 1712-1723.

## Claims

1. A method for the diagnosis of TB in a subject, the method comprising;
(a) providing a sample from said subject, said sample being selected from the group consisting of: blood, serum and plasma;
(b) determining the concentration in said sample of the following biomarkers: IL-1ra, IL6, IL-7, IL-8, IL-12p70, FGF-basic, IP-10, and VEGF;
(c) converting each biomarker concentration determined in (b) into a decile value; and
(d) converting each decile value into a binary presence or absence by comparing the decile values of (c) to the following specific quantile cut-off values:
| **Biomarker** | **Specific quantile cut-off value** |
|---|---|
| IL-1ra | 3 |
| IL6 | 6 |
| IL-7 | 8 |
| IL-8 | 9 |
| IL-12p70 | 9 |
| FGF-basic | 3 |
| IP-10 | 4 |
| VEGF | 9 |
wherein a decile value matching or exceeding the specific quantile cut-off value is converted into the binary presence of the biomarker, and a decile value lower than the specific quantile cut-off value is converted into the binary absence of the biomarker;
wherein detecting the presence of each of said biomarkers indicates that the subject has TB.

2. A method according to claim 1 wherein step (c) converting each concentration determined in (b) into a decile value comprises the steps of:
(ci) comparing the concentration of each biomarker determined in (b) to a reference frequency distribution of concentrations of said biomarker; and
(cii) reading out the decile value from the frequency distribution for the concentration of said biomarker.

3. A method according to claim 1 or claim 2 wherein determining the concentration of each biomarker comprises:
(bi) detection by contacting the sample with an antibody or antigen binding fragment thereof capable of specifically binding the biomarker; and
(bii) quantification of said binding.

4. A method according to claim 1 or claim 2 wherein determining the concentration of each biomarker comprises detection of the mRNA for the biomarker, wherein detection of the mRNA comprises:
(bi) contacting the sample with specific nucleic acid probe(s) or primer(s) for the biomarker; and
(bii) quantification of said probe(s) or primer(s).

5. A method according to any preceding claim wherein said sample is a sample of serum or plasma.

6. A method according to claim 5 wherein said serum or plasma is essentially cell free.

7. A method according to any preceding claim wherein the subject is 16 years old or younger.

8. A method according to any preceding claim wherein the subject is 15 years old or younger.

9. A method according to any preceding claim wherein the subject is 2 years old or older.

10. A method according to any preceding claim wherein the subject is 5 years old or older.

11. A method according to any preceding claim wherein the subject is 5 to 15 years old.

12. A method according to any preceding claim wherein said method further comprises determining the concentration in said sample of biomarker EC-stimulated VEGF (specific quantile cut-off value 2).

13. A process for selecting a treatment regimen, said process comprising carrying out the method according to any of claims 1 to 12, wherein if it is determined that the subject has TB then a treatment regimen of 2HRZE/4HR (2 months HRZE followed by 4 months HR wherein H = isoniazid, R = rifampicin, Z = pyrazinamide, E = ethambutol) is selected.

14. An apparatus comprising logic configured to carry out the method of any one of claims 1 to 12.

15. A computer program product operable, when executed on a computer, to perform the method steps of any one of claims 1 to 12.

## Patentansprüche

1. Verfahren zur Diagnose von TB bei einem Patienten, wobei das Verfahren Folgendes umfasst:
(a) Bereitstellen einer Probe von dem Patienten, wobei die Probe aus der Gruppe ausgewählt ist, bestehend aus: Blut, Serum und Plasma;
(b) Bestimmen der Konzentration der folgenden Biomarker in der Probe: IL-1ra, IL-6, IL-7, IL-8, IL-12p70, FGF-basisch, IP-10 und VEGF;
(c) Umwandeln von jeder in (b) bestimmten Biomarker-Konzentration in einen Dezilwert; und
(d) Umwandeln von jedem Dezilwert in eine binäre Anwesenheit oder Abwesenheit mittels Vergleich der Dezilwerte von (c) mit den folgenden spezifischen Quantil-Grenzwerten:
| **Biomarker** | **Spezifischer Quantil-Grenzwert** |
|---|---|
| IL-1ra | 3 |
| IL-6 | 6 |
| IL-7 | 8 |
| IL-8 | 9 |
| IL-12p70 | 9 |
| FGF-basisch | 3 |
| IP-10 | 4 |
| VEGF | 9 |
wobei ein Dezilwert, der dem spezifischen Quantil-Grenzwert entspricht oder darüber liegt, in die binäre Anwesenheit des Biomarkers umgewandelt wird, und ein Dezilwert, der unter dem spezifischen Quantil-Grenzwert liegt, in die binäre Abwesenheit des Biomarkers umgewandelt wird;
wobei der Nachweis der Anwesenheit von jedem der Biomarker anzeigt, dass der Patient an TB leidet.

2. Verfahren nach Anspruch 1, wobei Schritt (c), der jede in (b) bestimmte Konzentration in einen Dezilwert umwandelt, die folgenden Schritte umfasst:
(ci) Vergleich der Konzentration von jedem in (b) bestimmten Biomarker mit einer Bezugshäufigkeitsverteilung der Konzentrationen des Biomarkers; und
(cii) Auslesen des Dezilwerts aus der Häufigkeitsverteilung für die Konzentration des Biomarkers.

3. Verfahren nach Anspruch 1 oder Anspruch 2, wobei die Bestimmung der Konzentration von jedem Biomarker Folgendes umfasst:
(bi) Nachweis durch Inkontaktbringen der Probe mit einem Antikörper oder antigenbindenden Fragment davon, fähig zur spezifischen Bindung des Biomarkers; und
(bii) Quantifizierung der Bindung.

4. Verfahren nach Anspruch 1 oder Anspruch 2, wobei die Bestimmung der Konzentration von jedem Biomarker den Nachweis der mRNA für den Biomarker umfasst, wobei der Nachweis der mRNA Folgendes umfasst:
(bi) Inkontaktbringen der Probe mit einer spezifischen Nukleinsäuresonde oder einem spezifischen Nukleinsäureprimer oder spezifischen Nukleinsäuresonden oder spezifischen Nukleinsäureprimern für den Biomarker; und
(bii) Quantifizierung der Sonde(n) oder des Primers/der Primer.

5. Verfahren nach einem der vorangehenden Ansprüche, wobei die Probe eine Serum- oder Plasmaprobe ist.

6. Verfahren nach Anspruch 5, wobei das Serum oder Plasma im Wesentlichen zellfrei ist.

7. Verfahren nach einem der vorangehenden Ansprüche, wobei der Patient 16 Jahre alt oder jünger ist.

8. Verfahren nach einem der vorangehenden Ansprüche, wobei der Patient 15 Jahre alt oder jünger ist.

9. Verfahren nach einem der vorangehenden Ansprüche, wobei der Patient 2 Jahre alt oder älter ist.

10. Verfahren nach einem der vorangehenden Ansprüche, wobei der Patient 5 Jahre alt oder älter ist.

11. Verfahren nach einem der vorangehenden Ansprüche, wobei der Patient 5 bis 15 Jahre alt ist.

12. Verfahren nach einem der vorangehenden Ansprüche, wobei das Verfahren weiter die Bestimmung der Konzentration in der Probe vom EC-stimulierten VEGF-Biomarker (spezifisch Quantil-Grenzwert 2) umfasst.

13. Verfahren zur Auswahl eines Behandlungsregimes, wobei das Verfahren die Durchführung des Verfahrens nach einem der Ansprüche 1 bis 12 umfasst, wobei wenn bestimmt wird, dass der Patient an TB leidet, dann ein Behandlungsregime mit 2HRZE/4HR (2 Monate HRZE, gefolgt von 4 Monaten HR, wobei H = Isoniazid, R = Rifampicin, Z = Pyrazinamid, E = Ethambutol ist) ausgewählt wird.

14. Apparat, umfassend die Logik, konfiguriert zur Durchführung des Verfahrens nach einem der Ansprüche 1 bis 12.

15. Computerprogrammprodukt, das betriebsfähig ist, wenn es zur Durchführung der Verfahrensschritte nach einem der Ansprüche 1 bis 12 auf einem Computer laufen lassen wird.

## Revendications

1. Méthode de diagnostic de la TB chez un sujet, la méthode comprenant :
(a)l'obtention d'un échantillon chez ledit sujet, ledit échantillon étant sélectionné dans le groupe consistant en : le sang, le sérum et le plasma ;
(b)la détermination de la concentration en les biomarqueurs suivants dans ledit échantillon : IL-1ra, IL-6, IL-7, IL-8, IL-12p70, b-FGF, IP-10 et VEGF ;
(c)la conversion de la concentration en chaque biomarqueur déterminée en (b) en une valeur de décile ; et
(d)la conversion de chaque valeur de décile en une présence ou une absence binaire en comparant les valeurs de déciles de (c) aux valeurs seuils spécifiques des quantiles suivantes :
| **Biomarqueur** | **Valeur seuil spécifique du quantile** |
|---|---|
| IL-1ra | 3 |
| IL-6 | 6 |
| IL-7 | 8 |
| IL-8 | 9 |
| IL-12p70 | 9 |
| b-FGF | 3 |
| IP-10 | 4 |
| VEGF | 9 |
où une valeur de décile égale ou supérieure à la valeur seuil spécifique du quantile est convertie en la présence binaire du biomarqueur et une valeur de décile inférieure à la valeur seuil spécifique du quantile est convertie en l'absence binaire du biomarqueur ;
où la détection de la présence de chacun desdits biomarqueurs indique que le sujet est atteint de TB.

2. Méthode selon la revendication 1, où l'étape (c) de conversion de chaque concentration déterminée en (b) en une valeur de décile comprend les étapes de :
(ci) comparaison de la concentration en chaque biomarqueur déterminée en (b) à une distribution de référence des fréquences des concentrations en ledit biomarqueur ; et
(cii) lecture de la valeur de décile à partir de la distribution des fréquences pour la concentration en ledit biomarqueur.

3. Méthode selon la revendication 1 ou la revendication 2, où la détermination de la concentration en chaque biomarqueur comprend :
(bi) une détection par mise en contact de l'échantillon avec un anticorps ou un fragment de liaison à un antigène de celui-ci capable de se lier spécifiquement au biomarqueur ; et
(bii) une quantification de ladite liaison.

4. Méthode selon la revendication 1 ou la revendication 2, où la détermination de la concentration en chaque biomarqueur comprend la détection de l'ARNm du biomarqueur, où la détection de l'ARNm comprend :
(bi) une mise en contact de l'échantillon avec une/des sonde(s) ou amorce(s) d'acides nucléiques spécifiques du biomarqueur ; et
(bii) une quantification de la/des dite(s) sonde(s) ou amorce(s).

5. Méthode selon l'une quelconque des revendications précédentes, où ledit échantillon est un échantillon de sérum ou de plasma.

6. Méthode selon la revendication 5, où ledit sérum ou plasma est essentiellement exempt de cellules.

7. Méthode selon l'une quelconque des revendications précédentes, où le sujet est âgé de 16 ans ou moins.

8. Méthode selon l'une quelconque des revendications précédentes, où le sujet est âgé de 15 ans ou moins.

9. Méthode selon l'une quelconque des revendications précédentes, où le sujet est âgé de 2 ans ou plus.

10. Méthode selon l'une quelconque des revendications précédentes, où le sujet est âgé de 5 ans ou plus.

11. Méthode selon l'une quelconque des revendications précédentes, où le sujet est âgé de 5 à 15 ans.

12. Méthode selon l'une quelconque des revendications précédentes, où ladite méthode comprend en outre une détermination de la concentration dans ledit échantillon du biomarqueur qu'est le VEGF stimulé par EC (valeur seuil spécifique du quantile : 2).

13. Procédé de sélection d'un schéma thérapeutique, ledit procédé comprenant la réalisation de la méthode selon l'une quelconque des revendications 1 à 12 où, s'il est déterminé que le sujet est atteint de TB, un schéma thérapeutique consistant en 2HRZE/4HR (2 mois sous HRZE puis 4 mois sous HR, où H = isoniazide, R = rifampicine, Z = pyrazinamide, E = éthambutol) est alors sélectionné.

14. Appareil comprenant une logique conçue pour réaliser la méthode de l'une quelconque des revendications 1 à 12.

15. Produit-programme informatique utilisable, quand effectué sur un ordinateur, pour réaliser les étapes de la méthode de l'une quelconque des revendications 1 à 12.
